(19) Europäisches Patentamt
European Patent Office
Office européen des brevets

(11) EP 2 883 951 B1

(12) # EUROPEAN PATENT SPECIFICATION

(45) Date of publication and mention of the grant of the patent:
**17.01.2018 Bulletin 2018/03**

(21) Application number: **13805007.5**

(22) Date of filing: **14.06.2013**

(51) Int Cl.:
*C12N 1/04* (2006.01) *C12N 5/0735* (2010.01)
*C12N 5/0775* (2010.01) *C12N 5/0789* (2010.01)
*C12N 5/0797* (2010.01) *C12N 15/09* (2006.01)

(86) International application number:
**PCT/JP2013/003753**

(87) International publication number:
**WO 2013/187077 (19.12.2013 Gazette 2013/51)**

(54) **STEM CELL PRESERVATION MEDIUM, STEM CELL PRESERVATION METHOD, AND STEM CELL PRESERVATION SYSTEM**

MEDIUM ZUR STAMMZELLENKONSERVIERUNG, VERFAHREN ZUR STAMMZELLENKONSERVIERUNG UND SYSTEM ZUR STAMMZELLENKONSERVIERUNG

MILIEU DE CONSERVATION DE CELLULES SOUCHES, PROCÉDÉ DE CONSERVATION DE CELLULES SOUCHES, ET SYSTÈME DE CONSERVATION DE CELLULES SOUCHES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.06.2012 JP 2012136442**

(43) Date of publication of application:
**17.06.2015 Bulletin 2015/25**

(73) Proprietors:
- **Kyokuto Pharmaceutical Industrial Co. Ltd**
  **Chuo-Ku, Tokyo 103-0024 (JP)**
- **Riken**
  **Wako-shi, Saitama 351-0198 (JP)**

(72) Inventors:
- **AKUTA, Teruo**
  **Tokyo 103-0024 (JP)**
- **IMAIZUMI, Keitaro**
  **Tokyo 103-0024 (JP)**
- **NISHIKAWA, Shin-ichi**
  **Wako-shi**
  **Saitama 351-0198 (JP)**

(74) Representative: **Fleck, Hermann-Josef**
**Jeck & Fleck**
**Patentanwälte**
**Klingengasse 2**
**71665 Vaihingen/Enz (DE)**

(56) References cited:
**WO-A1-2011/021618 JP-A- 2006 238 875**
**JP-A- 2009 219 503 US-B1- 6 395 467**

- **A. STOLZING ET AL: "Hydroxyethylstarch in cryopreservation - Mechanisms, benefits and problems", TRANSFUSION AND APHERESIS SCIENCE, vol. 46, no. 2, 1 April 2012 (2012-04-01), pages 137-147, XP055241323, GB ISSN: 1473-0502, DOI: 10.1016/j.transci.2012.01.007**
- **R. MARTIN-IBANEZ ET AL: "Novel cryopreservation method for dissociated human embryonic stem cells in the presence of a ROCK inhibitor", HUMAN REPRODUCTION, vol. 23, no. 12, 1 December 2008 (2008-12-01), pages 2744-2754, XP055073464, ISSN: 0268-1161, DOI: 10.1093/humrep/den316**
- **KEITARO IMAIZUMI ET AL: "A Simple and Highly Effective Method for Slow-Freezing Human Pluripotent Stem Cells Using Dimethyl Sulfoxide, Hydroxyethyl Starch and Ethylene Glycol", PLOS ONE, vol. 9, no. 2, 12 February 2014 (2014-02-12), page e88696, XP055231301, DOI: 10.1371/journal.pone.0088696**
- **T'JOEN V. ET AL.: 'An Efficient, Economical Slow-Freezing Method for Large-Scale Human Embryonic Stem Cell Banking.' STEM CELLS AND DEVELOPMENT vol. 21, no. 5, 2012, pages 721 - 728**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 883 951 B1

- HOLM F. ET AL.: 'An effective serum- and xeno-free chemically defined freezing procedure for human embryonic and induced pluripotent stem cells.' HUMAN REPRODUCTION vol. 25, no. 5, 2010, pages 1271 - 1279
- BALCI D. ET AL.: 'The Assessment of Cryopreservation Conditions for Human Umbilical Cord Stroma-Derived Mesenchymal Stem Cells towards a Potential Use for Stem Cell Banking.' CURRENT STEM CELL RESEARCH & THERAPY vol. 8, no. 1, January 2013, pages 60 - 72

## Description

### [Technical Field]

The present invention relates to a preservation medium, a preservation method and a preservation system of stem cells, in particular, induced pluripotent stem cells (hereinafter, referred to as iPS cells) and embryonic stem cells (ES cells) of primates, and also relates to technologies for preserving simply and efficiently the cells not by means of vitrification but by means of slow-freezing preservation.

### [Background Art]

Industrial applications of human iPS/ES cells may include a field of regenerative medicine as well as a field of research and development of innovative drugs. An important point common to these fields is to cultivate and supply constant qualities of cells. In particular, human iPS/ES cells are low in survival rate after cryopreservation unlike murine cells, which poses a problem. Industrialization of human iPS/ES cells will require technologies of cryopreservation.

Cryopreservation of human iPS/ES cells is preceded by vitrification and slow-freezing methods. At the present time, in Japan, for example, in the iPS bank of research-use, BioResource
Center, Riken (the Institute of Physical and Chemical Research), a vitrification solution which is called DAP 213, is used to cryopreserve human iPS cells by means of vitrification. The DAP 213 is a vitrification solution developed for preserving fertilized ova of mice and high in solute concentrations, that is, 2 M of DMSO, 1 M of acetamide and 3 M of propylene glycol. The solution is quite high in toxicity due to osmotic pressure. Therefore, on freezing iPS/ES cells of primates including humans, it is necessary to treat the iPS/ES cells within a very short period of time, or about 10 seconds, from start of suspension to submersion into liquid nitrogen, and after being taken out from the liquid nitrogen on thawing, it is also necessary to rapidly thaw these cells by addition of a culture medium which has been in advance warmed at 37 °C. Further, these cells are not thawed in a state of being placed into a tube by using an incubator, which requires certain skills and experience.

In particular, in vitrification, water is not crystallized but solidified and frozen in a glass state. To that end, it is necessary to increase the concentration of a solute (cryoprotective agent) and enhance a cooling velocity. The higher the concentration of solute, the more easily vitrification can take place due to restricted molecular motions of water. However, the osmotic pressure is increased to result in an increase in toxicity of cells.

Further, re-crystallization will take place not on freezing but on thawing, thereby damaging the cells. And, this poses a serious problem.

In contrast, Ha SY and others reported a slow-freezing method for human iPS/ES cells in which human ES cells (SNUhES-3 strains) were subjected to cell dissociation by using a 0.25% (w/v) trypsin-0.53 mM EDTA solution and, thereafter a cryopreservation (5% DMSO, 50% FBS (fetal bovine serum), 10% EG, and 5% DMEM/F12) was slow-frozen and rapidly thawed to obtain a survival rate of 30.2%. (refer to Non-patent literature 1)

WuCF and others reported that a cryopreservation solution of human ES cells (hES 1 strain) which contained trehalose was slow-frozen and then rapidly thawed to obtain a survival rate of 48%. (refer to Non-patent literature 2)

Further, Holm F and others reported that human ES cell strains HS293, HS306 and human iPS cell strains (CHiPSA strains) were mechanically subjected to cell dissociation by using a cell scraper, thereafter, a unique cryopreservation solution, STEM-CELL BANKER (Registered trademark) (include DMSO, anhydrous dextrose and polymer), was used to effect slow freezing, then, on thawing, a unique thawing solution, CELLOTION (Registered trademark), was used to effect rapid thawing, thereby obtaining a survival rate of 90% to 90.6%. (refer to Non-patent literature 3)

T' Joen and others reported a similar technology in which a plurality of human ES cell strains were treated by the use of Collagenase IV, followed by Cell Dissociation Solution in two stages to effect cell dissociation so as to reduce the size of cell clumps, then, a cryopreservation solution (5% HES, 5% DMSO) and a preservation culture medium (80% DMEM/F12, 20% KSR and 400 μL HEPES) were used to effect slow freezing by a programmable freezer, thereafter, and a special recovery culture medium containing 0.1M sucrose was added on thawing to perform incubation for 5 minutes, thereby obtaining a survival rate of 80%. (refer to Non-patent literature 4)

Further they reported that where a cryopreservation solution (5% HES, and 5% or 10% EG) and a freezing culture medium (80% DMEM/F12, 20% KSR and 400 μL HEPES) were used to effect slow freezing to obtain a survival rate of 0% and also reported that although DMSO showed good recover rate, ethylene glycol alone provided no effective.

As described so far, although several reports are available about trials of slow-freezing methods in place of vitrification, they are not widely used as a general method. Reasons thereof include that these methods are low in reproducibility, not easily operated as compared with vitrification and need long working hours.

Further, in recent years, several groups reported a method in which Tryp LE (registered trade mark) Express or trypsin/EDTA was used to divide cells into single cells, the single cells were thereafter subjected to cryopreservation and cultivation was conducted by adding a ROCK inhibitor, Y27632, at a quantity of 10 μM to improve a survival rate (refer to Non-patent literatures 5 to 9) .

Thus, there has been desired the development of a robust and new cryopreservation method which is easier in handling, higher in efficiency, lower in toxicity and higher in reproducibility.

**[Prior Art]**

[Non-patent literature 1] Human Reproduction 20,1779-1785, 2005

[Non-patent literature 2] Reprod Biomed Online 11,733-739, 2005

[Non-patent literature 3] Human Reproduction 25,1271-1279 2010

[Non-patent literature 4] T' Joen et al., An efficient, economical slow-freezing method for large-scale human embryonic stem cell banking Stem Cells and Dev 2012 21:721-8

[Non-patent literature 5] Watanabe K etal.,Nat Biotechnol 25,681-686, 2007

[Non-patent literature 6] Martin-Ibanez R etal., Human Reprod 23,2744-2754,2008

[Non-patent literature 7] Martin-Ibanez R et al. , Human Reprod 24,492-493,2009

[Non-patent literature 8] Li XY etal., Human Reprod 24, 580-589, 2009

[Non-patent literature 9] Mollamohammadi S et al., Human Reprod 24, 2468-2476


**[Outline of the Invention]**

**[Problems to be Solved by the Invention]**

As described so far, vitrification requires a troublesome and complicated operation such as an increased concentration of a cryoprotective agent and a greater cooling velocity. On the other hand, slow-freezing methods tried in place of vitrification are low in cell survival rate and not simple in operation as compared with vitrification.

In view of the above-described situation, an object of the present invention is to provide a preservation medium of stem cells, a preservation method therefor and a preservation system thereof which can be used as a slow-freezing method in place of vitrification, high in cell survival rate, simple in operation and high in efficiency.

**[Means to Solve the Objects]**

The present inventor and others have diligently conducted research on preservation media for stem cells such as human iPS/ES cells by slow-freezing preservation and found that hydroxyethyl starch (HES), dimethyl sulfoxide (DMSO) and ethylene glycol (EG) are used in combination as a cryoprotective agent, thus making it possible to obtain a high cell survival rate.

They also have found that when cell dissociation is performance by a preservation method for slow-freezing preservation, a pronase solution is used to reduce the size of cell clumps and effect slow freezing, thereby obtaining a high cell survival rate.

That is, in order to attain the above-described object, the stem cell preservation medium of the present invention is a medium for preserving stem cells and characterized in containing hydroxyethyl starch (HES), dimethyl sulfoxide (DMSO) and ethylene glycol (EG) At the concentrations as defined in claim 1.

It is preferable that the stem cell preservation medium of the present invention contains hydroxyethyl starch (HES), dimethyl sulfoxide (DMSO) and ethylene glycol (EG) but does not contain any one of serum, a serum substitute component, a protein component (for example, albumin) or an animal derived component. In the case of what is called, an animal component-free stem cell preserving solution which does not contain any one of serum, a serum substitute component, a protein component such as albumin or an animal derived component, the solution is beneficially applicable to clinical studies of regenerative medicine in which human iPS/ES cells are used.

Further, the stem cell preservation medium of the present invention is a medium which can be preserved at normal temperature and, therefore, excellent in convenience.

As a cryoprotective agent, dimethyl sulfoxide (DMSO) is used in general. However, sole use of DMSO will decrease a survival rate of stem cells even where a step-wise freezing process is carried out by programmable freezing (cooling process down to ultra-low temperatures is programmed so as to effect automatic cooling and freezing) to store the stem cells in liquid nitrogen. Thus, CP-1 (manufactured by Kyokuto Pharmaceutical Industrial Co., Ltd.) known as a research reagent of cryoprotective agent for bone marrow and peripheral blood is used together with a mixed solution of hydroxyl ethylstarch (HES) and DMSO to improve a survival rate of stem cells. The CP-1 is composed of 12 g of HES and 10 mL of DMSO in a physiological saline solution. And, a human serum albumin solution and a RPMI 1640 culture medium are combined with CP-1 solution just before use. The CP-1 is characterized by being frozen and stored at -80°C and eliminating the necessity of programmable freezing.

The stem cell preservation medium of the present invention is such that ethylene glycol (EG) is added to a mixed solution of HES and DMSO, yielding a higher cell survival rate than CP-1. T' Joen and others reported in Non-Patent Document 4 that when only HES and EG were used, there were found no cryoprotective effects. The effect of the present invention is a new finding that high cell protective effects are obtained when HES, DMSO and EG are present at appropriate concentrations.

Further, where a cryotube preserved by slow freezing by using the stem cell preservation medium of the present invention is subjected to ultra-rapid thawing by addition of a warm culture medium or rapid thawing in a water bath (a hot-water bath kept at 37°C), there is obtained a higher cell survival rate than a case where other stem cell preservation media are used. In this case, a temperature of the warm culture medium is, for example, 20°C or higher, preferably 25°C or higher and more preferably 30°C or higher. The temperature is, for example, 40°C or lower, preferably 39°C or lower, more preferably 38°C or lower and most preferably about 37°C. Further, a high survival rate is obtained by addition of a normal culture medium without using a special recovery culture medium which contains a unique thawing solution, CELLOTIOM (registered trade mark) or 0.1M sucrose. No programmable freezing is needed, no special recovery culture medium is needed but the stem cell preservation medium of the present invention can be frozen and fused as with normal cell strains. Thus, the stem cell preservation medium is easy in operation, improved in robustness and beneficially applicable to clinical studies of regenerative medicine in which human iPS/ES cells are used.

In this case, ethylene glycol (EG) is present in a concentration range from 2% to 15% (v/v) and preferably from 4% to 12.5% (v/v). Where the concentration of EG is 2% or more and the cells are fused in a water bath (a hot-water bath kept at 37°C), a cell survival rate is in excess of 20%. Further, even where the concentration of EG is 15%, the cell survival rate is in excess of 20%. In particular, where the concentration of EG is within a range of 4% to 12.5% (v/v) and the cells are fused in a water bath (a hot-water bath kept at 37°C), there can be obtained a high cell survival rate as with rapid thawing by addition of a warm culture medium. Where the concentration of EG is less than 4% (v/v) or in excess of 12.5% (v/v), a cell survival rate is decreased. In addition, in view of the cell survival rate, the concentration of ethylene glycol (EG) is more preferably from 4 to 10% (v/v) and in particular preferably from 5% to 6% (v/v).

That is, where the three components of hydroxyethyl starch (HES), dimethyl sulfoxide (DMSO) and ethylene glycol (EG) are present at appropriate concentrations as defined in claim 1, there are exhibited excellent cell protective effects.

Further, empirically, hydroxyethyl starch (HES) is preferably present in a concentration range from 4% to 8% (w/v) and more preferably from 5% to 6% (v/v).

Since HES is a polysaccharide and will not permeate a cell membrane, it is effective in protecting a cell membrane from cell injury resulting from ice formation on a cell surface, which may contribute to improvement in cell survival rate after thawing.

Further, dimethyl sulfoxide (DMSO) is preferably from 4% to 6% (v/v) and more preferably at about 5% (v/v). DMSO will permeate the cell membrane and is effective in protecting a cell membrane from cell injury resulting from ice formation inside cells. However, DMSO has cytotoxicity, by which there are fears of inducing cell differentiation. The concentration of DMSO is reduced to 4% to 6% which is about half of 10%, which is a concentration at the time of cryopreservation in general.

Still further, ethylene glycol (EG) is added, thus making it possible to alleviate actions of inducing cell differentiation by DMSO. DMSO and ethylene glycol (EG) act as cryopreventive agents and suppress the formation of ice crystals on freezing. DMSO has been pointed out to have cell differentiation actions. Ethylene glycol (EG) is considered to counteract and decrease the actions.

In addition, rapid thawing by addition of a warm culture medium, dilution and centrifugal removal on thawing employed to reduce contact time with DMSO as much as possible, thus making it possible to suppress gene expression of a differentiation inducing gene as much as possible. In reality, confirmation has been made for markers of stem cells and in vitro three germ layer differentiation, by which retention of pluripotencies is confirmed.

Next, a description will be given of a stem cell preservation method of the present invention. The stem cell preservation method of the present invention is a preservation method for slow-freezing stem cells and provided with a dissociation step in which a pronase solution is used to dissociate the stem cells and a freezing step in which the dissociated stem cells are slow-frozen in a stem cell preservation medium.

In this case, the stem cell preservation medium is the said stem cell preservation medium of the present invention, that is, a medium which contains hydroxyethyl starch (HES), dimethyl sulfoxide (DMSO) and ethylene glycol (EG).

Alternatively, the stem cell preservation medium is a medium which contains hydroxyethyl starch (HES), dimethyl sulfoxide (DMSO) and a culture medium or an albumin solution, as with conventional CP-1.

In the stem cell preservation method of the present invention, treatment with a pronase solution is performed to finely divide cell colonies from which feeder cells have been removed into a certain size, thereafter, stem cells suspended in a buffer solution or a culture medium are mixed with a stem cell preservation medium as a cryopreservation agent and cooled. Cooling can be effected by slow freezing by using a programmable freezer or simple freezing process by using an ultra low-temperature freezer. In this case, the programmable freezer is used in a method of cooling rate by which the cells are planned-1°C to -2°C per minute. In contrast, the simple freezing process is such that cryotubes are placed in a foamed polystyrene box or a commercially available freezing container and also put into a freezer kept at -80°C and subjected to slow freezing at a velocity equal to that described above.

In the stem cell preservation method of the present invention, the simple freezing process can be used to perform work simply and efficiently.

In the dissociation step of the stem cell preservation method in the present invention, it is preferable that the size of clumps (dimension of cell aggregation) is in a range of 200 $\mu m^2$ to 10000 $\mu m^2$. This finding is empirically obtained by treatment of dissociating the stem cells with a pronase solution.

Further, in the freezing step of the stem cell preservation method in the present invention, it is preferable that the stem cells in a stem cell preservation medium are present in a range of 1 x $10^3$ to 1 x $10^6$ per mL of the preservation medium. A ratio of the stem cells which are subjected to cryopreservation to the stem cell preservation medium is within the said range, by which it is possible to attain a higher cell survival rate.

In the freezing step of the stem cell preservation method of the present invention, it is preferable that the stem cell preservation medium is approximately from 0.2 to 1 mL per cryotube.

In the stem cell preservation method of the present invention, ethylene glycol (EG) is present in a concentration range of 2% to 15% (v/v), preferably in the range of 4% to 12.5% (v/v). In view of the cell survival rate, ethylene glycol (EG) is more preferably present in a concentration range of 4% to 10% (v/v) and still more preferably present in the range of 5% to 6% (v/v).

Further, hydroxyethyl starch (HES) is preferably present in a concentration range of 4% to 8% (w/v) and more preferably present in the range of 5% to 6% (w/v). On the other hand, dimethyl sulfoxide (DMSO) is preferably present in a concentration range of 4% to 6% (v/v) and more preferably in the range of about 5% (v/v).

Further, in the stem cell preservation method of the present invention, it is preferable that the culture medium contains a medium selected from a group of culture media consisting of a Dulbecco modified Eagle (DMEM culture medium) and an F12 culture medium or it contains a mixture thereof.

Still further, in the stem cell preservation method of the present invention, it is preferable that an albumin solution is present at the concentration of about 4% (w/v).

In the stem cell preservation method of the present invention, the stem cell preservation medium is a medium which contains hydroxyethyl starch (HES), dimethyl sulfoxide (DMSO) and ethylene glycol (EG), and subjected to cryopreservation after the freezing step. Further, the freezing step includes a thawing step of effecting rapid thawing. It is preferable that in the thawing step, rapid thawing is performed by adding a warm culture medium to a cryotube. In the stem cell preservation method of the present invention, the stem cells which have been cryopreserved by using the said stem cell preservation medium of the present invention are able to attain a high cell survival rate, where the cryotube is fused in a water bath (a hot-water bath kept at 37°C).

Still further, it is preferable that there is included a cultivation step which conducts cultivation in a warm culture medium to which a ROCK inhibitor has been added, after the said thawing step. The ROCK inhibitor is added to conduct cultivation, thus making it possible to attain a higher cell survival rate than cultivation which is free of the ROCK inhibitor. In an application to clinical studies in which a greater emphasis is placed on safety and cost rather than efficiency, it is preferable to conduct cultivation without addition of the ROCK inhibitor. Cultivation by addition of the ROCK inhibitor is one of the important factors for attaining a high cell survival rate in a research reagent level. As the ROCK inhibitor, any given Rho-associated coiled-coil kinase (ROCK)-inhibiting substances are available, for example, Y-27632, Fasudil and H-1152 are illustrated.

In the said stem cell preservation method of the present invention, the stem cells are stem cells which are selected, for example, from a group consisting of tissue stem cells, embryonic stem (ES) cells and induced pluripotent stem (iPS) cells. Cells suitable for cryopreservation by using the stem cell preservation medium of the present invention include stem cells (in particular, human and primate induced pluripotent stem cells (iPS cells) and embryonic stem cells (ES cells)). It is acceptable that these cells are already-established cell strains or newly established cell strains.

Next, a description will be given of a stem cell preservation system of the present invention. The stem cell preservation system of the present invention is a preservation system for freezing stem cells and provided with a following 1) to 3).

1) a pronase solution as dissociation means for dissociating the stem cells
2) said stem cell preservation system of the present invention
3) slow freezing means for slow-freezing dissociated stem cells in the stem cell preservation medium

**[Effects of the Invention]**

The present invention is able to slow-freeze and preserve the stem cells simply and efficiently. Thus, it is possible to preserve the cells almost permanently, with the pluripotencies and proliferation potentials kept, and also to attain a high cell survival rate after thawing.

Further, according to the present invention, it is possible to attain a high cell survival rate and avoid an unstable cell survival rate in association with cryopreservation by conventional vitrification.

**[Brief Description of the Drawings]**

Fig. 1 shows survival rates of human iPS cells by using the cryopreservation solutions A to E according to the slow-freezing method.

Fig. 2 shows the results in comparison with the vitrification which uses DAP213 as a control experiment.

Fig. 3 shows the results of various survival rates obtained by preparing cryopreservation solutions with final concentrations of ethylene glycol (EG) from 1% to 15%.

Fig. 4 shows influences on the survival rate when various types of proteases are used in combination with a cryopreservation solution A.

Fig. 5 shows the results obtained by quantification of an area of clumps after treatment of cell dissociation by various types of proteases with reference to microscopic image data.

Fig. 6 shows the comparison of survival rates depending on whether the ROCK inhibitor is added or not.

Fig. 7 shows survival rates by the stem cell preservation method of the present invention upon use of the stem cell preservation medium containing HES, DMSO and EG in the human iPS cells 201B7 strains, the 253G1 strains and the reference example of the established human ES cell KhES1 strains.

Fig. 8 shows the results obtained by using main stem cell markers of human iPS/ES cells to perform a gene expression analysis and an immunocytochemical analysis by RT-PCR before and after freezing.

Fig. 9 shows the results in which after formation of embryoid bodies (EBs), human iPS cells were differentiated in vitro and main differentiation markers were used to performne expressionanm analysis and an immunocytochemical analysis by RT-PCR.

Fig. 10 is a flow chart of the stem cell preservation method in Embodiment 2.

Fig. 11 is a graph which shows substantially no difference in survival rate of human iPS cells 201B7 between batches of the cryopreservation solution A after freezing and thawing.

Fig. 12 is a graph which shows no change in proliferation potentials of the human iPS cells 201B7 before and after freezing and thawing.

Fig. 13 is shows that chromosomes of the human iPS cells 201B7 strains after freezing and thawing are normally retained.

**[Best Mode for Carrying Out the Invention]**

Embodiments of the present invention will be described in detail below with reference to the drawings. The present invention is not limited to the following embodiment and examples of shown in the figure, and the present invention can be variously changed in design.

**[Embodiment 1]**

In one embodiment of the stem cell preservation medium of the present invention, 6g of hydroxyethyl starch (HES), 5 mL of dimethyl sulfoxide (DMSO) and 5 mL of ethylene glycol (EG) are dissolved in 100 mL of physiologically saline solution, thereby producing a stem cell preservation medium.

Next, the stem cells suspended in a buffer solution and a culture medium are subjected to treatment with a pronase solution to divide fine cell colonies from which feeder cells have been removed to a predetermined size. Thereafter, cell pellets after centrifugation of the stem cells are mixed with the said stem cell preservation medium. After mixture of the stem cell preservation medium with the stem cells, a mixture thereof is cooled to -80°C at a velocity of 1 to 2°C per minute and subjected to cryopreservation. Cooling is carried out by placing a cell suspension housed in a protective case in a foamed polystyrene box inside a freezer kept at -80°C. In addition, the cell suspension may be further cooled down to a temperature of liquid nitrogen and stored in the liquid nitrogen.

Thawing can be conducted rapidly by procedures in which a culture medium kept warmed in advance at 37°C is added to ice floe so as to be diluted 10 times or more or by procedures in which the culture medium is submerged into warm water kept at 37°C. More specifically, frozen cells are placed into a thermostatic chamber kept at 37 to 40°C, and rapidly thawed, while being well agitated. It is desirable that 0.5 mL of the cell suspension in a cryotube is thawed within 2 to 3 minutes as a target.

**[Embodiment 2]**

Consideration has been made for a cell survival rate (hereinafter, referred to as survival rate) after cryopreservation of human iPS cells in the stem cell preservation method of the present invention.

As described above, the stem cell preservation method of the present invention is provided with a dissociation step which dissociates stem cells by using a pronase solution and a freezing step in which the dissociated stem cells are slow-frozen in a stem cell preservation medium.

Further, the stem cell preservation medium as a cryopreservation solution to be used is a medium which contains hydroxyethyl starch (HES), dimethyl sulfoxide (DMSO) and ethylene glycol (EG) or a medium which contains hydroxyethyl starch (HES), dimethyl sulfoxide (DMSO) and a culture medium or an albumin solution.

In the Example, the pronase solution used was a pronase solution for cell dispersion (manufactured by Kyokuto Pharmaceutical Industrial Co., Ltd.).

Further, five types below of cryopreservation solutions were prepared. Concentrations listed below are based on final concentrations.

(Cryopreservation solution A) 6% HES, 5% DMSO, 5% EG and 0.9% NaCl
(Cryopreservation solution B) 6% HES, 5% DMSO, 4% BSA, 50% DMEM/F12 and 0.9% NaCl
(Cryopreservation solution C) 6% HES, 5% DMSO, 4% BSA and 0.9% NaCl
(Cryopreservation solution D) 6% HES, 5% DMSO, 50% DMEM/F12 and 0.9% NaCl
(Cryopreservation solution E) 6% HES, 5% DMSO and 0.9% NaCl

The said BSA is a 25% bovine serum albumin solution (obtained by dissolving albumin supplied from Sigma Inc., in a physiologically saline solution manufactured by Otsuka Pharmaceutical Co. , Ltd.). Further, the said DMEM/F12 is a culture medium obtained by mixing a Dulbecco modified Eagle (DMEM culture medium) with an F12 culture medium.

The cryopreservation solutions A to E were prepared by using a cryoprotective solution CP-1 (manufactured by Kyokuto Pharmaceutical Industrial Co., Ltd., 551-27200-0, 68 mL of physiologically saline solution containing 12 g of HES and 10 mL of DMSO).

More specifically, the cryopreservation solution A was prepared by procedures in which 3.2 mL of physiologically saline solution was added to 6.8 mL of CP-1 stock solution to give 10 mL, a resultant thereof was well mixed and, thereafter, a physiologically saline solution containing 10% EG (manufactured by Wako Pure Chemical Industries, Ltd.) was gently mixed therewith in an equivalent quantity of 10 mL at a low temperature.

Further, the cryopreservation solution B was prepared by procedures in which 3.2 mL of 25% BSA was added to 6.8 mL of CP-1 stock solution to give 10 mL, a resultant thereof was well mixed and, thereafter, a DMEM/F12 culture medium was gently mixed therewith in an equivalent quantity of 10 mL at a low temperature.

Further, the cryopreservation solution C was prepared by procedures in which 3.2 mL of 25% BSA solution was added to 6.8 mL of CP-1 stock solution to give 10 mL, a resultant thereof was well mixed and, thereafter, a physiologically saline solution was gently mixed therewith in an equivalent quantity of 10 mL at a low temperature.

Still further, the cryopreservation solution D was prepared by procedures in which 3.2 mL of a physiologically saline solution was added to 6.8 mL of CP-1 stock solution to give 10 mL, a resultant thereof was well mixed and, thereafter, a DMEM/F12 culture medium was gently mixed therewith in an equivalent quantity of 10 mL at a low temperature.

In addition, the cryopreservation solution E was prepared by procedures in which 3.2 mL of physiologically saline solution was added to 6.8 mL of CP-1 stock solution to give 10 mL and a resultant thereof was gently mixed therewith at a low temperature.

Human iPS cells 201B7 strains, 253G1 strains or human ES cells KhES1 strains and H1 strains (HhES1 and H1 strains disclosed for reference only) were cultivated in accordance with a human iPS cells retaining cultivation protocol (RIKEN Center for Developmental Biology, Human Stem Cell Technology Unit). That is, the culture medium to which were added 80% DMEM/F12 (with Glutamax but without HEPES), 1% non-essential amino acid, 20% serum replacement KSR (products described so far were manufactured by Invitrogen Corporation), 100 IU/mL of penicillin, 100 μg/mL of streptomycin (manufactured by Meiji Seika Pharma Co., Ltd.) and, immediately prior to use, also added were 0.1 mM of 2-melcaptoethanol (manufactured by Wako Pure Chemical Industries, Ltd.) and 5 ng/mL of bFGF (manufactured by Wako Pure Chemical Industries, Ltd.).

Further, feeder cells were prepared by procedures in which SNL cells (manufactured by DS Pharma Biomedical Co., Ltd.) were treated in advance by using mitomycycin C (manufactured by Kyowa Hakko Kirin Co., Ltd.) at final concentrations of 10 μg/mL to stop the growth and the cells were seeded on the previous day on a plate consisting of 3 x $10^5$ cells/well/6 well and a cultivation plate which was coated with 0.1% gelatin type A (manufactured by Sigma Inc.)

solution. The culture medium was changed every other day, and passage cultivation was conducted, with one passage being four days. On passage, the cells were dissociated by using a CTK dissociation solution of human ES cells (1 mg/mL of Collagenase IV + 0.25% Trypsin + 20% KSR + 1mM of $CaCl_2$/PBS) ($CaCl_2$ manufactured by Nacalai Tesque, Inc. and others manufactured by Invitrogen Corporation), and passage cultivation was conducted in a 1:6 dilution ratio.

In slow freezing, the cells were washed by using 2 to 3 mL of a phosphate-buffered saline PBS (-) except for a culture medium of human iPS cells which was in a confluent state. 1 mL of a pronase solution for cell dispersion was added thereto and a resultant was treated at 37°C for about one minute, and only the feeder cells were in advance dissociated, floated and removed by aspiration. Immediately, washing was performed by using PBS (-) which was then removed by aspiration, and a culture medium, 2 mL, was added thereto. The cells were dissociated and harvested by gentle pipetting by the use of a P1000 pipette and stained with trypan blue to count the number of cells. The cells of about 2.5 x 104 were dispensed into a 1.5 mL tube and, thereafter, subjected to centrifugation (300g, for 3 minutes at 4°C) from which supernatant was removed as much as possible, and cell pellets were prepared. Said cryopreservation solutions A to E were added to the prepared cell pellets at the respective quantities of 0.5 mL and transferred to cryotubes, with lids covered thereon. These tubes were transferred to a freezing container (Nalgene), and frozen overnight at -80°C. On the following day, the tubes were transferred to a deep freezer kept at -150°C and subjected to cryopreservation at least for 7 days.

Thawing was conducted in accordance with two protocols, that is, thawing process 1 and thawing process 2 given below.

a) Thawing process 1: rapid thawing by dilution in which a warm culture medium which has been preincubated is directly added to a vial (dilution thawing by addition of warm culture medium)
More specifically, the tubes are taken out from a deep freezer kept at -150°C and rapidly thawed in 5 mL of a culture medium which has been in advance preincubated at 37°C, transferred to a 15 mL centrifugation tube (to which 3 mL of the culture medium is in advance added) and subjected to centrifugation (300g, for 3 minutes at 4°C), from which supernatant is removed by aspiration.
b) Thawing process 2: thawing in a water bath
More specifically, the tubes are taken out from a deep freezer kept at -150°C and subjected to rapid thawing in a water bath kept at 37°C, and 5 mL of a culture medium is added thereto. Thereafter, a resultant thereof is transferred to a 15 mL centrifugation tube (3 mL of the culture medium is in advance added) and subjected to centrifugation (300g, for 3 minutes at 4°C) from which supernatant is removed by aspiration.

A new culture medium containing 10μM of Y-27632 was addded to the cell pellets harvested by said thawing operation of a) or b), a resultant thereof was subjected to on-feeder cultivation on a 6-well plate. Cultivation was carried out for 48 hours in a ROCK inhibitor-containing culture medium, and after the culture medium was changed, cultivation was further carried out in a ROCK inhibitor-free culture medium for 3 days (a total of 5 days).

Fig. 10 is a flow chart which shows a stem cell preservation method in this Example. The flow chart of Fig. 10 shows a stem cell preservation method where the thawing process 1 (dilution thawing by addition of a warm culture medium) is used. More specifically, as shown in Fig. 10, a pronase solution is used to dissociate the stem cells (dissociation step: Step S01) and, then, the thus dissociated stem cells are subjected to slow freezing (freezing step: Step S03) in a stem cell preservation medium to effect cryopreservation (Step S05). The stem cell preservation medium used in the freezing step is any one of said cryopreservation solutions A to E.

Upon thawing of the stem cells which have been subjected to cryopreservation, a warm culture medium is added to the cryotubes (ultra-rapid thawing) or the stem cells are rapidly fused in a water bath kept at 37 °C and, thereafter, they are centrifuged to quickly remove a freezing medium (thawing step: Step S07), cultivation is carried out in a ROCK inhibitor-containing culture medium (up to 48 hours). Thereafter, cultivation is carried out in a normal culture medium (cultivation step: Step S09). Next, large-scale cultivation (Step S11) is carried out or the process returns to the dissociation step (Step S01) to resume cryopreservation.

In addition, on thawing, both of rapid thawing and ultra-rapid thawing can be employed. In particular, a rapid thawing process at which thawing is conducted in a water bath kept at 37°C is the same as the thawing process for ordinary cell strains. This process is advantageous in being fewer in operation mistakes and higher in robustness.

Comparison was also made for vitrification which used DAP213, as a control experiment. The cells were washed by using 2 to 3 mL of PBS (-), except for a culture medium of human iPS cells which was in a confluent state and, thereafter, treatment was performed at 37°C for about one minute by adding 1 mL of CTK, and only feeder cells were in advance dissociated, floated and removed by aspiration. The cells were immediately washed by using PBS (-) and removed by aspiration. 5 mL of a culture medium was added thereto and a resultant was gently pipetted by using a P1000 pipette, and 500μL of the resultant (about 2.5 x $10^5$/tube) was dispensed into a 1.5 mL tube so as not to collapse clumps. After centrifugation (300g, for 3 minutes at 4°C), supernatant was removed as much as possible, and DAP213 freezing solution (2 M DMSO (manufactured by Calbiochem Inc.), 1 M acetamide (manufactured by Sigma Inc.) and 3 M proplylene glycol (manufactured by Nacalai Tesque, Inc.), 0.2 mL were added to a cell pellet. The cell pellet was rapidly frozen within 10 seconds by using liquid nitrogen and transferred to liquid nitrogen or a deep freezer kept at -150°C and subjected to cryopreservation for 7 days.

After cultivation, staining with alkaline phosphatase (ALP) was carried out by procedures in which a culture medium in the 6 well plate was removed by aspiration, the cells were washed with PBS (-) (pH 7. 4) (manufactured by Invitrogen Corporation) three times, fixed with PBS (-) containing 4% paraformaldehyde (manufactured by Nacalai Tesque, Inc.) at room temperature for 15 minutes, washed three times with PBS (-), thereafter, there was added 1 mL of ALP stain solution which was prepared by sequentially adding Reagent-1, Reagent-2 and Reagent-3 of ALP Substrate Kit IV (manufactured by Vector Laboratory Inc. , SK-5400,) at one drop each in relation to 2.5 mL of 100 mM Tris-HCl (pH 9.5), and the thus-prepared cells were allowed to stand at room temperature until a black color developed. The number of colonies was counted on the basis of digital images (4x) obtained by an imaging system (manufactured by Keyence Corporation).

The following formula was used to calculate a survival rate.

Calculation was made on the basis of survival rate (%) = number of colonies at 5-day cultivation after thawing/the number of colonies at 5-day cultivation after passage x 100.

Fig. 1 shows the results. Fig. 1 is a graph which shows survival rates of human iPS cells by using the cryopreservation solutions A to E according to the slow-freezing method.

Where the thawing process 1 (dilution thawing by addition of a warm culture medium) was used to effect thawing, a survival rate obtained by using the cryopreservation solution A was 88.4%, a survival rate obtained by using the cryopreservation solution B was 96.4%, a survival rate obtained by using the cryopreservation solution C was 94.3%, a survival rate obtained by using the cryopreservation solution D was 76.3%, and a survival rate obtained by using the cryopreservation solution E was 88.1% (each of the survival rates was a median value).

On the other hand, where the thawing process 2 (thawing in a water bath) was used to effect thawing, a survival rate obtained by using the cryopreservation solution A was 78.7%, a survival rate obtained by using the cryopreservation solution B was 44.5%, a survival rate obtained by using the cryopreservation solution C was 47.3%, a survival rate of the cryopreservation solution D was 33.5%, and a survival rate obtained by using the cryopreservation solution E was 38.5% (each of the survival rates was a median value) .

As shown in Fig. 1, where the thawing process 1 (dilution thawing by addition of a warm culture medium) was carried out to thaw the human iPS cells according to the slow-freezing method by using said cryopreservation solutions A to E, all of the survival rates was 75% or higher. As a result, it has been found that the human iPS cells can be subjected to cryopreservation. In particular, it has been found that the cells can be subjected to cryopreservation where there were used the cryopreservation solutions A, D, E diluted with a physiologically saline solution or a culture medium which is free of BSA.

Further, as shown in Fig. 1, where the thawing process 2 (thawing in a water bath) was carried out to effect thawing according to the slow-freezing method by using the cryopreservation solutions A to E, the survival rate was 75% or higher only when the cryopreservation solution A was used. It has been found that a high survival rate is obtained, irrespective of which thawing process is carried out when the slow-freezing method is carried out by using the cryopreservation solution A.

Further, Fig. 2 shows the results in comparison with the vitrification which uses DAP213 as a control experiment. As shown in Fig. 2, where the cells were subjected to cryopreservation by the vitrification using DAP213 and the thawing process 1 (dilution thawing by addition of a warm culture medium) was carried out to effect thawing, the survival rate was 80% or higher. Where the thawing process 2 (thawing in a water bath) was carried out to effect thawing, the survival rate was zero.

Still further, Fig. 11 shows a comparison experiment between batches where the slow-freezing method was carried out by using the cryopreservation solution A. It has been found that the survival rate is high and not influenced by a difference between batches, irrespective of which thawing process is carried out.

As described so far, it has been found that where the cells were dissociated by using a pronase solution, slowly frozen by using the cryopreservation solutions A to E and thawed by the thawing process 1, the cells exhibited a survival rate of 80% or higher.

It has been found that, in particular, where the slow-freezing method was carried out by using the cryopreservation solution A (the stem cell preservation medium of the present invention), the survival rate was 80% or higher, irrespective of which thawing process is carried out. The cryopreservation solution A (the stem cell preservation medium of the present invention) is an animal component-free cryopreservation solution, that is, a solution which does not contain BSA or a culture medium, and beneficially applicable to practical clinical studies of regenerative medicine which uses human iPS cells.

[Embodiment 3]

In Embodiment 3, for the purpose of searching an optimal concentration of ethylene glycol (EG), there were prepared cryopreservation solutions with final concentrations of ethylene glycol (EG) ranging from 1% to 15% on the basis of the cryopreservation solution A of Embodiment 2 (the stem cell preservation medium of the present invention), and comparison was made for individual survival rates.

Fig. 3 shows the results thereof. As shown in Fig. 3, consideration was made for cryopreservation solutions with various concentrations of ethylene glycol (EG) to find that a solution with 5% ethylene glycol (EG) showed a survival rate of 90% or higher, irrespective of which thawing process is carried out.

Where the thawing process 1 of Embodiment 2 was carried out, with no ethylene glycol (EG) being added, a survival rate was low or 14.5%. Where the concentration of ethylene glycol (EG) was from 5% to 10%, the survival rate was improved. However, the survival rate was again decreased at the concentration of 12.5% or higher.

Where no ethylene glycol (EG) was added and the concentration of ethylene glycol (EG) was from 2% to 5% in the thawing process 2 of Embodiment 2, the survival rate was 80% or higher. Where the concentration of ethylene glycol (EG) was 5%, the survival rate was 102% and improved to the greatest extent. At the concentration of ethylene glycol (EG) of 4% to 10%, the survival rate was stable and recovered, irrespective of which thawing process is carried out.

With consideration given to convenience for operators and robustness of a protocol, these results indicate that a survival rate can be kept high either by the thawing in a water bath or the rapid thawing where the concentration of ethylene glycol (EG) is 5%. And the protocol is robust and not influenced by a working speed.

**[Embodiment 4]**

In Embodiment 4, the stem cell preservation method of the present invention is carried out by using a pronase solution to dissociate stem cells, showing that a higher survival rate is obtained by cryopreservation than cell dissociation by using various types of proteases.

More specifically, the stem cells were dissociated by using the following cell dissociation solutions of five types of proteases and subjected to cryopreservation by using the cryopreservation solution A of Embodiment 2 (the stem cell preservation medium of the present invention). Thereby, the stem cells were fused and cultivated by being rapidly thawed by additon of a water bath culture medium to consider an optimal combination.

(Cell dissociation solution A) pronase solution, at 37°C, for one minute
(Cell dissociation solution B) 0.05% Trypsin/EDTA (manufactured by Invitrogen Corporation), at 37°C, for one minute
(Cell dissociation solution C) 2 mg/mL of Disease II (manufactured by Roche Inc. , 10x stocks are diluted with DMEM/F12), at 37°C, for fifteen minutes
(Cell dissociation solution D) 1.5 mg/mL of Collagenase IV (powder is dissovled with DMEM/F12), at 37°C, for forty minutes
(Cell dissociation solution E) CTK, at 37°C, for one minute

Fig. 4 shows the results. It is apparent from Fig. 4 that pronase is optimal.

In addition, in Fig. 4 and Fig. 5, Pronase indicates the cell dissociation solution A; Trypsin indicates the cell dissociation solution B; Disease indicates the cell dissociation solution C; Collagenase indicates the cell dissociation solution D; and CTK indicates the cell dissociation solution E.

Further, on the basis of the dimension (the size of clumps) of a cell aggregation on cell dissociation obtained by using various types of proteases, the 201B7 strain was used to quantify a subsequent size of clumps.

After cell dissociation, the cells were harvested by using a conventional culture medium, a suspension solution thereof was placed into a 6-well plate to photograph a digittal image (4x) by using an imaging system (manufactured by Keyence Corporation), and a quantification was made for the size of clumps by using an area determination mode of accessory software.

Fig. 5 shows the results thereof. Where the cells were dissociated by using a pronase solution (pronase treatment), the size of clumps was 2089 $\pm$ 1485 $\mu m^2$. This size was significantly small ($P < 0.01$) as compared with 25454 $\pm$ 41002 $\mu m^2$ on dispase treatment and 8551 $\pm$ 18884 $\mu m^2$ on collagenase treatment and 8600 $\pm$ 13267 $\mu m^2$ on CTK treatment and also smaller in variation. On the other hand, where trypsin treatment was carried out, the size of clumps was 1936 $\pm$ 1663 $\mu m^2$, which was similar to that on pronase treatment, and no significant difference was found.

Further, on treatment with pronase and trypsin which produced a small size of clumps, the cells were able to be cryopreserved after dissociation in the respective dissociation solutions.

However, where the survival rate obained on pronase treatment was compared with that obtained on trypsin treatment, it was found that the survival rate on pronase treatment was 88%, while, that on trypsin treatment was 30%. The survival rate on pronase treatment was significantly higher.

Superiority of pronase treatment to trypsin treatment may be due to a) and b) reasons given below.

a) Unknown protein necessary for survival of iPS cells on the surface of cells was decomposed and protein for inducing apoptosis exerted influences.

b) Protease of trypsin was greater in efficacy than pronase, inactivation after action was difficult, and the same iPS cells were partially divided into single cells due to remaining activity of protease by pipetting operation or movement between tubes, after determination of the size of colonies. In particular, human iPS cells in an epistem state are high in survival rate in a certain state of cell clump size. However, when divided into single cells, these cells were unable to survive unlike cells derived from an inner cell mass in a ground state, for example, mice ES cells.

On the other hand, in the other three types of proteases which produced a greater size of clumps on treatment, large colonies found on passage were unable to survive after cryopreservation and only small colonies survived in a very small probability.

Reduction in size of clumps is crucial for success of cryopreservation. According to cell dissociation by using a pronase solution in the stem cell preservation method of the present invention, there are advantages that the iPS cells can be separated from feeder cells in a short period of time and a colony can be finely divided as well as these cells are high in survival rate and free of animal derived components.

**[Embodiment 5]**

In Embodiment 5, a description will be given of a difference in survival rate between ROCK inhibitor (+) cultivation and ROCK inhibitor (-) cultivation in the stem cell preservation method of the present invention.

Cultivation and freezing in the stem cell preservation method were carried out by procedures similar to those of Embodiment 2. Regarding thawing, after a warm culture medium was used to conduct rapid thawing, difference of survival rate was measured depending on whether the ROCK inhibitor 10μM Y-27632 was added or not. Where the inhibitor was added, in the presence of Y-27632, cultivation was performed for two days, thereafter, cultivation was performed additionally for three days in a culture medium free of Y-27632. On the other hand, where no inhibitor was added, cultivation was performed for five days in a normal culture medium. On both the cultivation, a survival rate was measured according to the method described in Embodiment 2.

As shown in Fig. 6, the measurement showed that on ROCK inhibitor (+) cultivation, a survival rate was about 120% or the survival rate higher than that of about 30% on ROCK inhibitor (-) cultivation. As described so far, it has been found that the ROCK inhibitor (+) is an important reagent for supporting a high survival rate in the stem cell preservation method.

In the future, when consideration is given to applications to clinical studies such as regenerative medicine, a ROCK inhibitor-free culture medium is preferable in terms of safety, step management and cost. In Fig. 5, on ROCK inhibitor (-) cultivation, a survival rate was about 30%, thereby confirming the survival of the cells in the absence of the inhibitor. ROCK inhibitor (-) cultivation is applicable to clinical studies in which a greater emphasis is placed on the safety and cost rather than efficiency.

**[Embodiment 6]**

In Embodiment 6, a description will be given of effectiveness of the stem cell preservation method of the present invention which uses a stem cell preservation medium which contains hydroxyethyl starch (HES), dimethyl sulfoxide (DMSO) and ethylene glycol (EG) on treatment of established human iPS cell 201B7 strains, 253G1 strains, established human ES cell KhES1 strains and H1 strains (KhES1 and H1 strains disclosed for reference only).

Fig. 7 shows the results thereof. High survival rates from 80% to 100% were confirmed in the human iPS cells 201B7 strains, the 253G1 strains, the established human ES cell KhES1 strains and the H1 strains (KhES1 and H1 strains disclosed for reference only). That is, it has been found that the stem cell preservation method of the present invention which uses the stem cell preservation medium containing HES, DMSO and EG is effective in cryopreservation of a plurality of stem cells strains.

**[Embodiment 7]**

Embodiment 7 shows the results obtained by performing a semi-quantitative analysis of gene expression by RT-PCR (Reverse Transcriptase - Polymerase Chain Reaction) for the presence or absence of change in gene expression in a state of human iPS cells before and after freezing. Table 1 shows a primer list for RT-PCR gene expression analysis. A request was made for Invitrogen Corporation, to synthesize the primer with reference to literature (Takenaka et al. Experimental Hematology. 2010. 38(2):154-62.).

[Table 1]

| Gene | Primer | Sequence |
|---|---|---|
| OCT4 endogenous | Forward | GACAGGGGGAGGGGAGGAGCTAGG |
| | Reverse | CTTCCCTCCAACCAGTTGCCCCAAAC |
| KLF4 endogenous | Forward | GATTACGCGGGCTGCGGCAAAACCTACACA |
| | Reverse | TGATTGTAGTGCTTTCTGGCTGGGCTCC |
| SOX2 endogenous | Forward | GGGAAATGGGAGGGGTGCAAAAGAGG |
| | Reverse | TTGCGTGAGTGTGGATGGGATTGGTG |
| C-MYC endogenous | Forward | TTTCTGAAGAGGACTTGTTGCGGAAACGAC |
| | Reverse | TCAGCCAAGGTTGTGAGGTTGCATTTGATC |
| NANOG | Forward | CAGCCCCGATTCTTCCACCAGTCCC |
| | Reverse | CGGAAGATTCCCAGTCGGGTTCACC |
| GAPDH | Forward | CTTCTTTTGCGTCGCCAGCCGAG |
| | Reverse | CAGCCTTGACGGTGCCATGGAA |

Regarding iPS cells before freezing (Day 4 after passage) and after thawing (Day 7), a portion of the iPS cells covering 3 wells of the 6-well plate was dissociated by using a CTK solution and the thus dissociated iPS cells were harvested by centrifugation. Total RNA was prepared in accordance with a protocol of an RN easy mini kit (manufactured by QIAGEN Inc.). More specifically, the cells were dissolved in 600 μL of RLT Buffer (2ME was added at a quantity of 1/100 immediately before dissolution) and a resultant was stored at -80°C. Thereafter, on-column DNase treatment (at room temperature for 15 minutes) was carried out by using a QIAshredder (manufactured by QIAGEN Inc.), RNase-free DNase I kit (manufactured by QIAGEN Inc.), and the resultant was finally dissolved in 50 μL of RNase-free Water.

The concentration of RNA was determined by measurement of absorbance at 260 nm by using a Nanodrop (manufactured by Thermo Fisher Scientific K.K.). Reverse transcription reactions were conducted by procedures in which 2 μL of 5x RT Buffer, 0.5 μL of RT Enzyme Mix and 0.5 μL of Primer Mix were added to 100 ng of Total RNA which was modified by rapid cooling after heat treatment at 65°C for 5 minutes according to a protocol attached to a Rever Tra Ace qPCR RT kit (manufactured by Toyobo Co., Ltd.) to give 10 μL of a system and reverse transcription reactions were carried out at 37°C for 15 minutes, then, heat treatment was carried out at 98 °C for 5 minutes in the system to deactivate enzymes, thereby preparing cDNA.

The thus obtained cDNA was diluted ten times by addition of 90 μL of RNase-free Water, thereby giving a cDNA solution. Polymerase Chain Reactions (PCR) were carried out by the procedures in which, with 1 μL of the cDNA solution being given as a mold, in a total system of 10 μL, 1 μL of 10x ExTaq Buffer, 0.8 μL of 2.5 mM dNTP, 0.05 μL of ExTaq HS (all manufactured by Takara Bio Inc.) and 1 μL of 10 μM primer mix were used to conduct the reactions at 30 cycles for 10 seconds at 98°C, for 20 seconds at 55°C and for 30 seconds at 72°C, and the reactions were carried out for another 5 minutes at 72°C. 10x Loading Buffer was added at each quantity of 1μL to an entire quantity of reaction solution and the entire quantity of the reaction solution was subjected to electrophoresis for 10 minutes at a constant voltage of 120V by using 2% agarose gel. After staining with ethidium bromide, detection was performed by UV.

Further, immunocytochemical analysis was made for pluripotency markers (OCT4, SSEA3, SSEA4, TRA-1-60 and TRA-1-81) of the human iPS cells before and after freezing by using fluorescent-labeled antibodies. More specifically, human iPS cells 201B7 strains were subjected to on-feeder cultivation for 5 days on a 24-well plate and a culture medium was removed by aspiration, thereafter, PBS (-) containing 4% paraformaldehyde was added at a quantity of 500 μL/well, and the thus treated strains were allowed to stand at room temperature for 15 minutes and fixed. A fixative solution was removed by aspiration therefrom and washing was performed three times by using about 2 mL of PBS (-) (for five minutes). Only on detection of OCT4, 1 mL of ice-cold methanol was added and this marker was subjected to penetration treatment at -20°C for 10 minutes, followed by one time washing with PBS. The other markers were allowed to stand at room temperature for 60 minutes by using a blocking solution (5% goat serum, 0.3% Triton X-100/PBS (-)) and subjected to blocking.

After removal of a blocking solution, as a primary antibody, anti-OCT 4 mice monoclonal antibody (manufactured by Santa Cruz Inc.), anti-SSEA3 rat monoclonal antibody (manufactured by Millipore Inc.), anti-SSEA4 mice monoclonal antibody (manufactured by CST Inc.), anti-TRA-1-60 mice monoclonal antibody (manufactured by CST Inc.) and anti-TRA-1-81 mice monoclonal antibody (manufactured by CST Inc.) were all used so as to be diluted 200 times by using an antibody dilution solution (PBS (-) containing 1% BSA and 0.3% Triton X-100) and allowed to stand at 4 °C overnight, thereby allowing reactions to proceed. Subsequently, about 2 mL of PBS (-) was used to effect washing (for 5 minutes) three times. As a secondary fluorescent-labeled antibody, Alexa Fluoro 488-labeled anti-mice IgG goat antibody, Alexa Fluoro 488-labeled anti-mice IgM goat antibody, Alexa Fluoro 488-labeled anti-mice IgM goat antibody (all manufactured by Invitrogen Corporation) were similarly diluted 500 times by an antibody dilution solution and added thereto. Resultants thereof were blocked for light by using aluminum foil and allowed to react at room temperature for 2 hours.

Next, the cells were washed once by using PBS containing 0.4M NaCl and allowed to stand at room temperature for 5 minutes, with light being blocked, after addition of a DAPI solution (manufactured by Invitrogen Corporation), a stock solution was diluted 1000 times by using PBS (-)), thereby performing nuclear staining. The cells were washed three times for 5 minutes by using about 2 mL of PBS (-) and observed under fluorescent light by using an image system (manufactured by Keyence Corporation).

Fig. 8 shows the results thereof. As shown in Fig. 8(1), main stem cells markers (OCT4, KLF4, SOX2, C-MYC and NANOG) for human iPS/ES cells (human iPS cells 201B7, reference human ES cells KhES-1) were analyzed by RT-PCR to find bands. Thereby, no conspicuous difference in gene expression was confirmed before and after freezing. Further, as shown in Fig. 8(2), since the main stem cells markers (OCT4, SSEA3, SSEA4, TRA-1-60 and TRA-1-81) were found to be stained in all cases by immunocytochemical analysis, no conspicuous difference was confirmed before and after freezing.

**[Embodiment 8]**

Embodiment 8 shows the results obtained by consideration in vitro three germ layer differentiation.

After freezing and thawing by the stem cell preservation medium of the present invention which contains hydroxyethyl starch (HES), dimethyl sulfoxide (DMSO) and ethylene glycol (EG), the 201B 7 strains were subjected to on-feeder cultivation for 7 days, from which SNL cells were removed with a CTK dissociation solution. Thereafter, the 201B7 strains were recovered by using a human iPS cells culture medium (-bFGF) and cultivated for 10 days on a low attachment surface plate (manufactured by Corning Incorporated), thereby forming embryoid bodies (EBs). The culture medium was changed every other day.

A half quantity of the embryoid bodies (EBs) was recovered into a tube, and a three germ layer-specific marker of was used to perform a gene expression analysis by RT-PCR. More specifically, an RLT Buffer (included in a RN easy mini kit manufactured by Qiagen GmbH) was used to extract RNA, and RT (reverse transcription) and PCR were conducted according to normal procedures. Table 2 shows a primer list for gene expression analysis by RT-PCR.

[Table 2]

| | Gene | Primer | Sequence |
|---|---|---|---|
| Ectoblast | PAX6 | Forward | ACCCATTATCCAGATGTGTTTGCCCGAG |
| | | Reverse | ATGGTGAAGCTGGGCATAGGCGGCAG |
| Mesoblast | BRACHYURY | Forward | GATGCAGTGACTTTTTGTCG |
| | | Reverse | CTACTGCAGGTGTGAGCAAG |
| Hypoblast | SOX17 | Forward | CGCTTTCATGGTGTGGGCTAAGGACG |
| | | Reverse | TAGTTGGGGTGGTCCTGCATGTGCTG |
| | GATA4 | Forward | CTAGACCGTGGGTTTTGCAT |
| | | Reverse | TGGGTTAAGTGCCCCTGTAG |
| Reference | GAPDH | Forward | CTTCTTTTGCGTCGCCAGCCGAG |
| | | Reverse | CAGCCTTGACGGTGCCATGGAA |

A remaining half quantity of the embryoid bodies (EBs) was seeded and attached on a gelatin-coated 24-wells plate, followed by 4-day cultivation in a human iPS cells culture medium (-bFGF). Thereafter, primary antibodies and secondary antibodies of three germ layer-specific markers given in Talbe 3 below were used to stain the embryoid bodies. Next, nuclei thereof were stained with DAPI and detected by using a fluorescence imaging system (manufactured by Keyence Corporation). Table 3 covers the antibodies used in fluorescence immune antibody staining.

[Table 3]

| Antibody | Number | Product name | Company | Catalog No. | Secondary antibody used |
|---|---|---|---|---|---|
| Primary antibody (200 times dilution) | (1) | Monoclonal Anti-b-Tubulin Clone TUB 2.1 | SIGMA | T4026 | (4) |
| | (2) | Monoclonal Anti-Actin, α-Smooth Muscle antibody produced in mouse | SIGMA | A2547 | (5) |
| | (3) | AFP (3H8) Mouse mAb | CST | #3903 | (5) |
| Secondary antibody (500 times dilution) | (4) | Alexa Fluor® 546 goat anti-mouse IgG | Invitrogen | A11003 | |
| | (5) | Alexa Fluor® 488 goat anti-mouse IgG | Invitrogen | A11001 | |

Fig. 9 shows the results thereof. As shown in Fig. 9(1), the human iPS cells 201B7 strains were subjected to in vitro differentiation after formation of embryoid bodies (EBs), and main differentiation markers (ectoderm PAX6, mesoderm BRACHYURY, endoderm SOX17 and GATA4) were analyzed by RT-PCR to confirm the bands, thereby confirming the gene expression of differentiation genes. Further, as shown in Fig. 9(2), main differentiation cell markers (ectoderm $\beta$-tubulin, mesoderm $\alpha$ SMA, and endoderm $\alpha$ Fetoprotein (AFP)) were stained in all cases by immunocytochemical analysis. As a result, it was confirmed that three germ layer differentiation potentials were retained.

**[Embodiment 9]**

In Embodiment 9, a description will be given of the effectiveness and stability of the stem cell preservation method of the present invention which uses a stem cell preservation medium to confirm that human iPS cells are not changed in proliferation potentials before and after freezing and thawing.

Before freezing, the established human iPS cells 201B7 strains were dissociated from a 6-well plate having an initial seeding concentration of about 25,000/well every four days by using CKT and passaged in a 1 : 6 dilution ratio. After thawing, an aliquot of 25,000/vial was cultivated on a 10-cm across dish for one week, dissociated every four days by using CTK and passaged in a 1:6 dilution ratio. Cell numbers were counted microscopically with a hemocytometer by procedures in which sampling was performed with the lapse of time, and the cells were divided into single cells with 0.05 Trypsin/EDTA treatment (at 37°C for 10 minutes), followed by staining with trypan blue.

Fig. 12 shows the results. It was confirmed that the established human iPS cells 201B7 strains were substantially constant in the number of cells before and after freezing and thawing.

**[Embodiment 10]**

In Embodiment 10, a description will be given of the effectiveness and safety of the stem cell preservation method of the present invention which uses a stem cell preservation medium to confirm that chromosomes after freezing and thawing are retained normally in human iPS cells.

Fig. 13 shows the results thereof. Established human iPS cells 201B7 strains during 3 to 5 passages of cultivation after thawing were subjected to analysis of G-bands, thereby confirming their normal karyotype.

A description has been so far given of the present invention. In addition, Japanese Patent Application No. 2012-136422 filed on June 15, 2012, is to be incorporated into the specification as it is with reference. Further, all publication, patents and patent applications cited in the present specification are to be incorporated as they are into the specification.

**[Industrial Applicability]**

The present invention is effectively applicable to research of cellular biology of stem cells (in particular, human iPS cells and reference ES cells and fields of regenerative medicine such as organ transplantation using cell banks of stem cells and cells differentiated from stem cells.

## Claims

1. A stem cell preservation medium which is a medium for preserving stem cells and which contains hydroxyethyl starch (HES), dimethyl sulfoxide (DMSO) and ethylene glycol (EG), wherein
ethylene glycol (EG) is present in a concentration range of 4% to 12.5% (v/v), hydroxyethyl starch (HES) is present in a concentration range of 4% to 8% (w/v), and
dimethyl sulfoxide (DMSO) is present in a concentration range of 4% to 6% (v/v).

2. The stem cell preservation medium according to Claim 1 which is free of any one of serum, a substitute component of serum, a protein component and an animal-derived component.

3. A stem cell preservation method which is a preservation method for slow-freezing stem cells, comprising a dissociation step for dissociating the stem cells by using a pronase solution; and
a freezing step for slow-freezing the thus dissociated stem cells in a stem cell preservation medium according to Claim 1, wherein
there is additionally included a thawing step for conducting rapid thawing after a freezing step, and
the thawing step is to effect thawing in a water bath to a cryotube.

4. The stem cell preservation method according to Claim 3, wherein the size of clumps is from 200 $\mu m^2$ to 10000 $\mu m^2$

in the dissociation step.

**5.** The stem cell preservation method according to Claim 3, wherein stem cells of the stem cell preservation medium are present in a range of 1 x 103 to 1 x 106 per mL of the preservation medium in the freezing step.

**6.** The stem cell preservation method according to Claim 5, wherein the stem cell preservation medium is approximately 0.2 mL to 1 mL per cryotube in the freezing step.

**7.** The stem cell preservation method according to Claim 3, wherein the culture medium contains a culture medium which is selected from a group consisting of a Dulbecco modified Eagle medium (DMEM culture medium) and an F12 culture medium or a mixture thereof.

**8.** The stem cell preservation method according to Claim 3, wherein an albumin solution is present at the concentration of approximately 4% (w/v).

**9.** The stem cell preservation method according to Claim 3, wherein there is additionally included a cultivation step in which after the thawing step, cultivation is performed in a warm culture medium to which a ROCK inhibitor has been added.

**10.** The stem cell preservation method according to Claim 3, wherein the stem cells are stem cells selected from a group consisting of tissue stem cells, embryonic stem (ES) cells and induced pluripotent stem (iPS) cells.

**11.** A stem cell preservation system which is a system for preserving stem cells, and
the stem cell preservation system, comprising:

a pronase solution as dissociation means for dissociating the stem cells;
a stem cell preservation medium according to Claim 1; and
slow freezing means for slow-freezing said dissociated stem cells in the stem cell preservation medium.

**12.** The stem cell preservation medium according to Claim 1 which is used for preserving stem cells from which the stem cells have been dissociated by using a pronase solution.

**Patentansprüche**

**1.** Stammzellenkonservierungsmedium, das ein Medium zum Konservieren von Stammzellen ist und das Hydroxyethylstärke (HES), Dimethylsulfoxid (DMSO) und Ethylenglykol (EG) enthält, wobei
Ethylenglykol (EG) in einem Konzentrationsbereich von 4 % bis 12,5 % (v/v) vorliegt,
Hydroxyethylstärke (HES) in einem Konzentrationsbereich von 4 % bis 8 % (w/v) vorliegt, und
Dimethylsulfoxid (DMSO) in einem Konzentrationsbereich von 4 % bis 6 % (v/v) vorliegt.

**2.** Stammzellenkonservierungsmedium nach Anspruch 1, das frei von jedem aus einem Serum, einer Substitutionskomponente eines Serums, einer Proteinkomponente und einer vom Tier abgeleiteten Komponente ist.

**3.** Stammzellenkonservierungsverfahren, das ein Konservierungsverfahren für langsam gefrierende Stammzellen ist, umfassend
einen Dissoziationsschritt zum Dissozüeren der Stammzellen unter Verwendung einer Pronasenlösung; und
einen Gefrierschritt zum langsamen Einfrieren der so dissoziierten Stammzellen in einem Stammzellenkonservierungsmedium nach Anspruch 1,
wobei es einen zusätzlich enthaltenen Auftauschritt zum schnellen Auftauen nach einem Gefrierschritt gibt, und
wobei der Auftauschritt beschaffen ist, Auftauen in einem Wasserbad zu einem Kryoröhrchen zu bewirken.

**4.** Stammzellenkonservierungsverfahren nach Anspruch 3, wobei die Größe der Klumpen in dem Dissoziationsschritt von 200 $\mu m^2$ bis 10000 $\mu m^2$ beträgt.

**5.** Stammzellenkonservierungsverfahren nach Anspruch 3, wobei Stammzellen des Stammzellenkonservierungsmediums in einem Bereich von 1 x 103 bis 1 x 106 pro ml des Konservierungsmediums in dem Gefrierschritt vorliegen.

6. Stammzellenkonservierungsverfahren nach Anspruch 5, wobei das Stammzellenkonservierungsmedium in dem Gefrierschritt annähernd 0,2 ml bis 1 ml pro Kryoröhrchen beträgt.

7. Stammzellenkonservierungsverfahren nach Anspruch 3, wobei das Kulturmedium ein Kulturmedium enthält, das ausgewählt ist aus einer Gruppe bestehend aus einem Dulbecco-modifizierten Eagle-Medium (DMEM-Kulturmedium) und einem F12-Kulturmedium oder einem Gemisch daraus.

8. Stammzellenkonservierungsverfahren nach Anspruch 3, wobei eine Albuminlösung in einer Konzentration von etwa 4 % (w/v) vorliegt.

9. Stammzellenkonservierungsverfahren nach Anspruch 3, wobei es einen zusätzlich enthaltenen Kultivierungsschritt gibt, in dem nach dem Abtauschritt die Kultivierung in einem warmen Kulturmedium durchgeführt wird, zu dem ein ROCK Inhibitor hinzugefügt worden ist.

10. Stammzellenkonservierungsverfahren nach Anspruch 3, wobei die Stammzellen Stammzellen sind, die ausgewählt sind aus einer Gruppe bestehend aus Gewebestammzellen, embryonalen Stammzellen (ES) und induzierten pluripotenten Stammzellen (iPS).

11. Stammzellenkonservierungssystem, das ein System zum Konservieren von Stammzellen ist, und wobei das Stammzellenkonservierungssystem umfasst:

eine Pronaselösung als Dissoziationsmittel zum Dissozüeren der Stammzellen;
ein Stammzellenkonservierungsmedium nach Anspruch 1; und
langsam-Gefriermittel zum langsamen Einfrieren der dissoziierten Stammzellen in dem Stammzellenkonservierungsmedium.

12. Stammzellenkonservierungsmedium nach Anspruch 1, das verwendet wird, um Stammzellen zu konservieren, aus denen Stammzellen unter Verwendung einer Pronaselösung dissozüert wurden.

## Revendications

1. Milieu de conservation de cellules souches, lequel est un milieu destiné à conserver des cellules souches et lequel contient de l'hydroxyéthylcellulose (HEC), du diméthylsulfoxyde (DMSO) et de l'éthylèneglycol (EG), où l'éthylèneglycol (EG) est présent selon une plage de concentration comprise entre 4 % et 12,5 % (v/v), l'hydroxyéthylcellulose (HEC) est présente selon une plage de concentration comprise entre 4 % et 8 % (m/v), et le diméthylsulfoxyde (DMSO) est présent selon une plage de concentration comprise entre 4 % et 6 % (v/v).

2. Milieu de conservation de cellules souches selon la revendication 1, lequel est exempt de tout sérum, de tout composant de sérum de substitution, de tout composant protéique et de tout composant issu d'un animal.

3. Procédé de conservation de cellules souches, lequel est un procédé de conservation destiné à réaliser une congélation lente de cellules souches, comprenant une étape de dissociation destinée à dissocier les cellules souches en utilisant une solution de pronase ; et
une étape de congélation destinée à congeler lentement les cellules souches ainsi dissociées dans un milieu de conservation de cellules souches selon la revendication 1, où
une étape de décongélation destinée à réaliser une décongélation rapide après une étape de congélation est également incluse, et
l'étape de décongélation consiste à effectuer une décongélation dans un bain d'eau sur un cryotube.

4. Procédé de conservation de cellules souches selon la revendication 3, dans lequel la taille des amas est comprise entre 200 $\mu m^2$ et 10 000 $\mu m^2$ lors de l'étape de dissociation.

5. Procédé de conservation de cellules souches selon la revendication 3, dans lequel les cellules souches du milieu de conservation de cellules souches sont présentes selon une plage comprise entre 1 x 103 et 1 x 106 par ml du milieu de conservation lors de l'étape de congélation.

6. Procédé de conservation de cellules souches selon la revendication 5, dans lequel le volume de milieu de conservation de cellules souches est approximativement compris entre 0,2 ml et 1 ml par cryotube lors de l'étape de congélation.

**7.** Procédé de conservation de cellules souches selon la revendication 3, dans lequel le milieu de culture contient un milieu de culture lequel est sélectionné parmi le groupe constitué d'un milieu Eagle modifié de Dulbecco (milieu de culture DMEM) et d'un milieu de culture F12 ou d'un mélange de ceux-ci.

**8.** Procédé de conservation de cellules souches selon la revendication 3, dans lequel une solution d'albumine est présente à une concentration d'environ 4 % (m/v).

**9.** Procédé de conservation de cellules souches selon la revendication 3, dans lequel une étape de culture est également incluse, lors de laquelle, après l'étape de décongélation, une culture est réalisée dans un milieu de culture chaud auquel un inhibiteur de kinase ROCK a été ajouté.

**10.** Procédé de conservation de cellules souches selon la revendication 3, dans lequel les cellules souches sont des cellules souches sélectionnées parmi le groupe constitué de cellules souches tissulaires, de cellules souches embryonnaires (CSE) et de cellules souches pluripotentes induites (CSPi).

**11.** Système de conservation de cellules souches, lequel est un système destiné à conserver des cellules souches, et le système de conservation de cellules souches, comprenant :

une solution de pronase en tant que moyen de dissociation destiné à dissocier les cellules souches ;
un milieu de conservation de cellules souches selon la revendication 1 ; et
un moyen de congélation lente destiné à congeler lentement lesdites cellules souches dissociées dans le milieu de conservation de cellules souches.

**12.** Milieu de conservation de cellules souches selon la revendication 1, lequel est utilisé pour conserver des cellules souches à partir desquelles les cellules souches ont été dissociées en utilisant une solution de pronase.

Fig.1

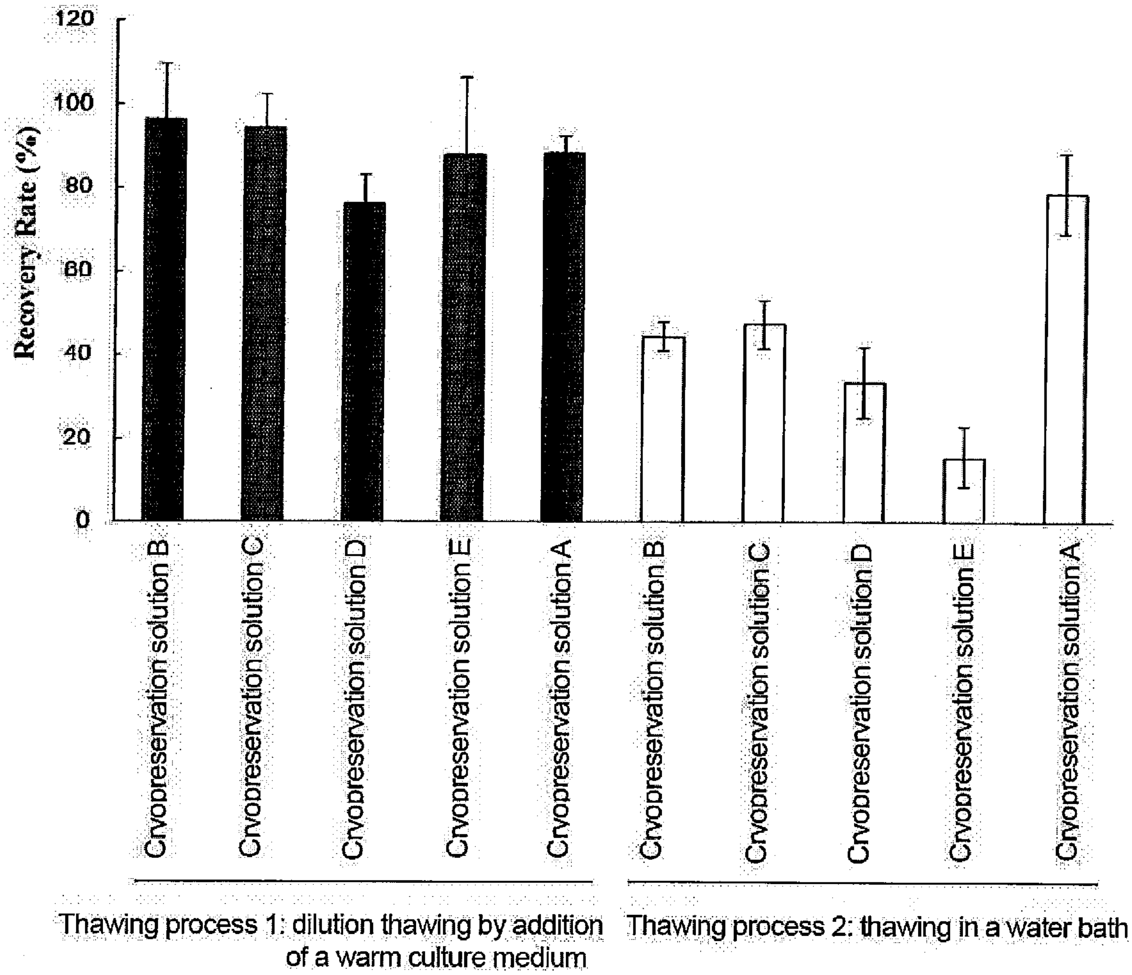


(Cryopreservation solution A) 6% HES, 5% DMSO, 5% EG and 0.9% NaCl

(Cryopreservation solution B) 6% HES, 5% DMSO, 4% BSA, 50% DMEM/F12 and 0.9% NaCl

(Cryopreservation solution C) 6% HES, 5% DMSO, 4% BSA and 0.9% NaCl

(Cryopreservation solution D) 6% HES, 5% DMSO, 50% DMEM/F12 and 0.9% NaCl

(Cryopreservation solution E) 6% HES, 5% DMSO and 0.9% NaCl

Fig.2

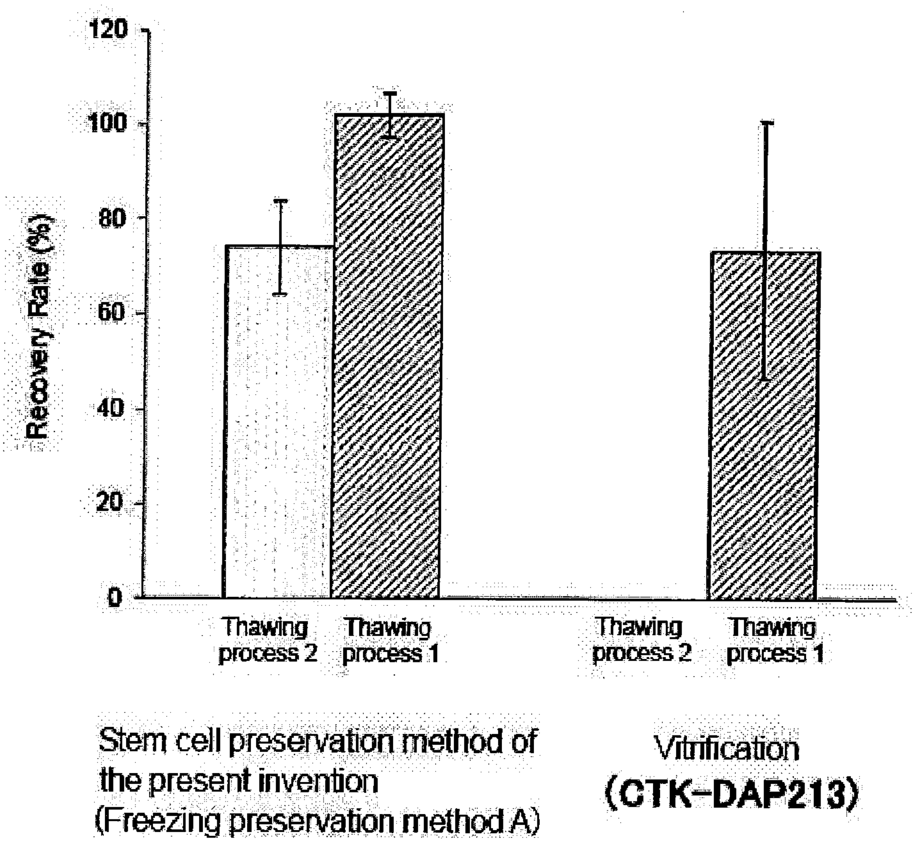
120
100
80
60
40
20
0
Recovery Rate (%)
Thawing process 2
Thawing process 1
Thawing process 2
Thawing process 1
Stem cell preservation method of the present invention (Freezing preservation method A)
Vitrification (CTK-DAP213)

Fig.3

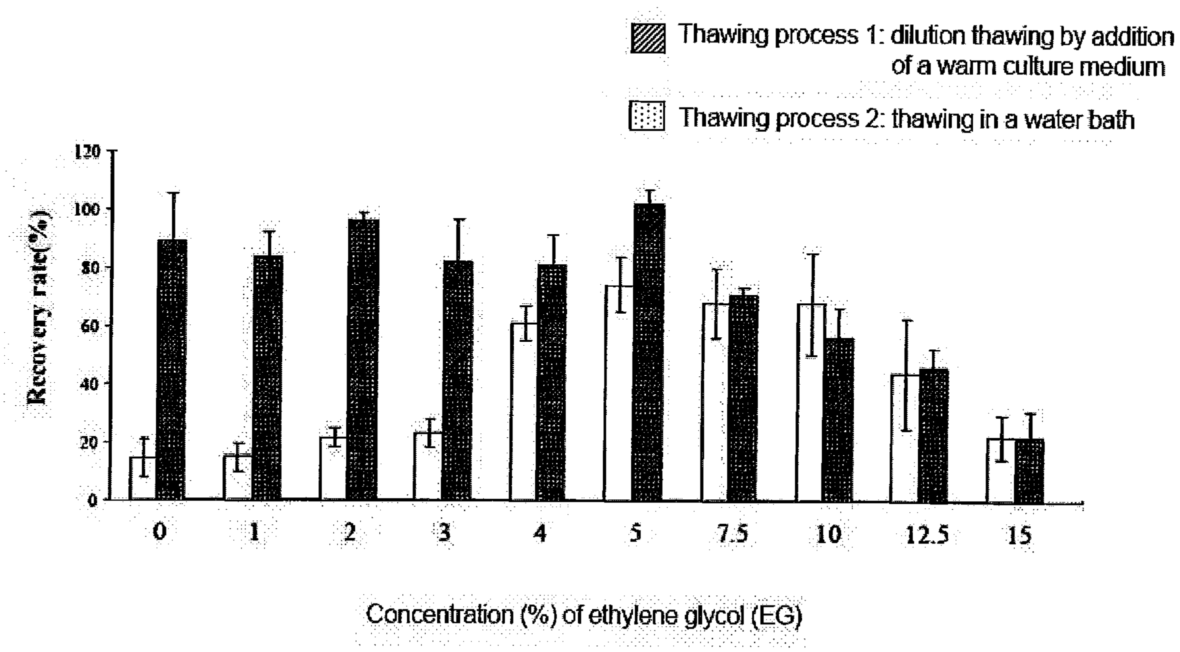
Thawing process 1: dilution thawing by addition of a warm culture medium
Thawing process 2: thawing in a water bath
120
100
80
60
40
20
0
Recovery rate(%)
0
1
2
3
4
5
7.5
10
12.5
15
Concentration (%) of ethylene glycol (EG)

Fig.4

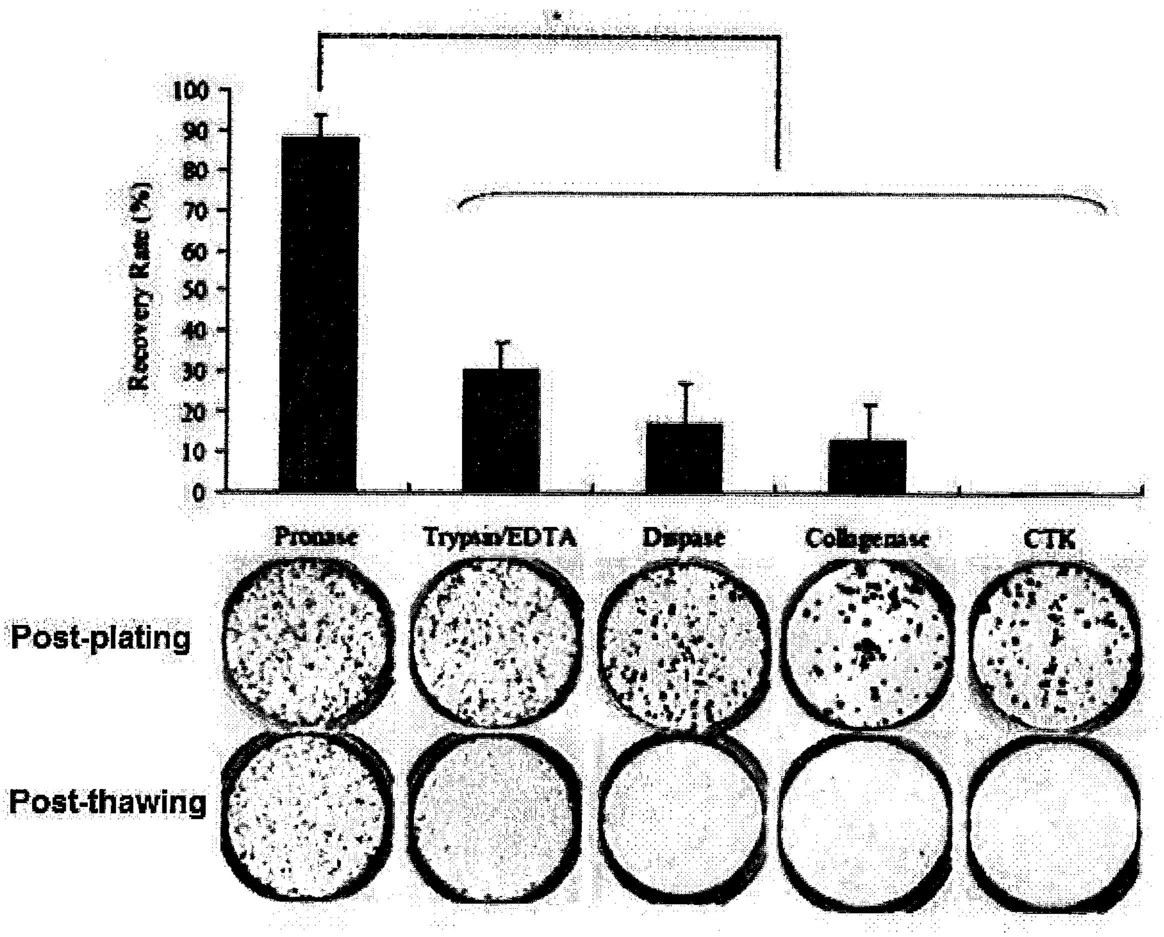
*
100
90
80
70
60
50
40
30
20
10
0
Recovery Rate (%)
Pronase
Trypsin/EDTA
Dispase
Collagenase
CTK
Post-plating
Post-thawing

Fig.5

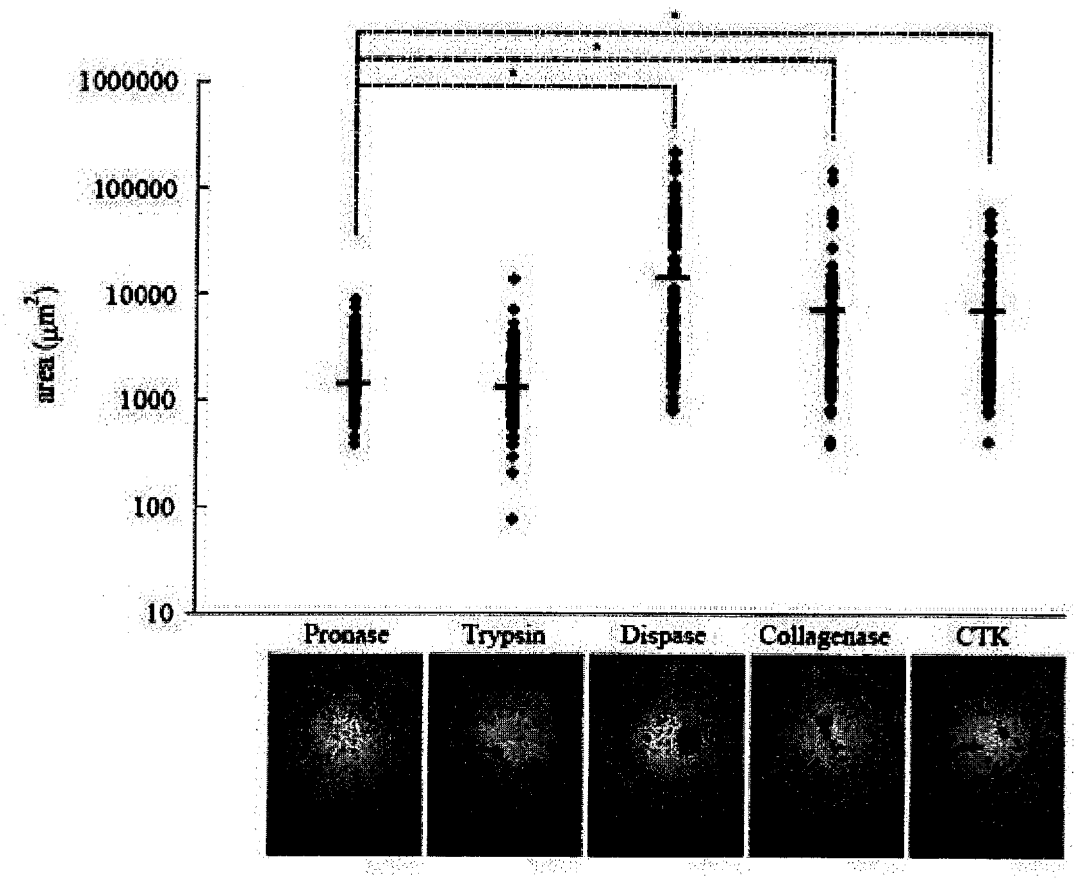
1000000
100000
10000
1000
100
10
area (μm2)
Pronase
Trypsin
Dispase
Collagenase
CTK
*
*
*

Fig.6

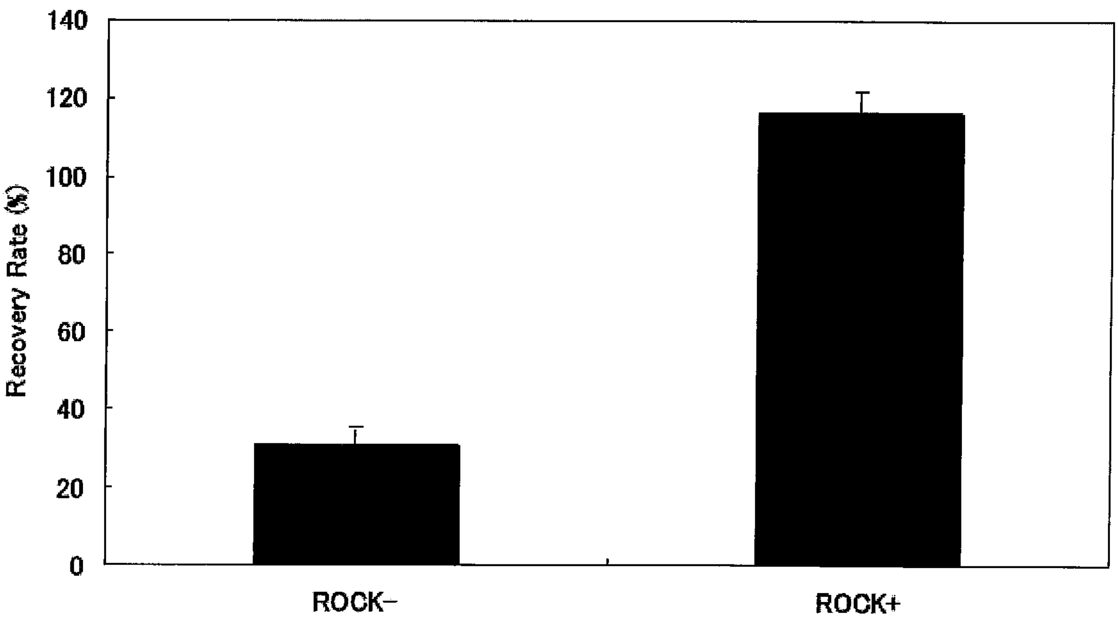
140
120
100
80
60
40
20
0
Recovery Rate (%)
ROCK−
ROCK+

Fig.7

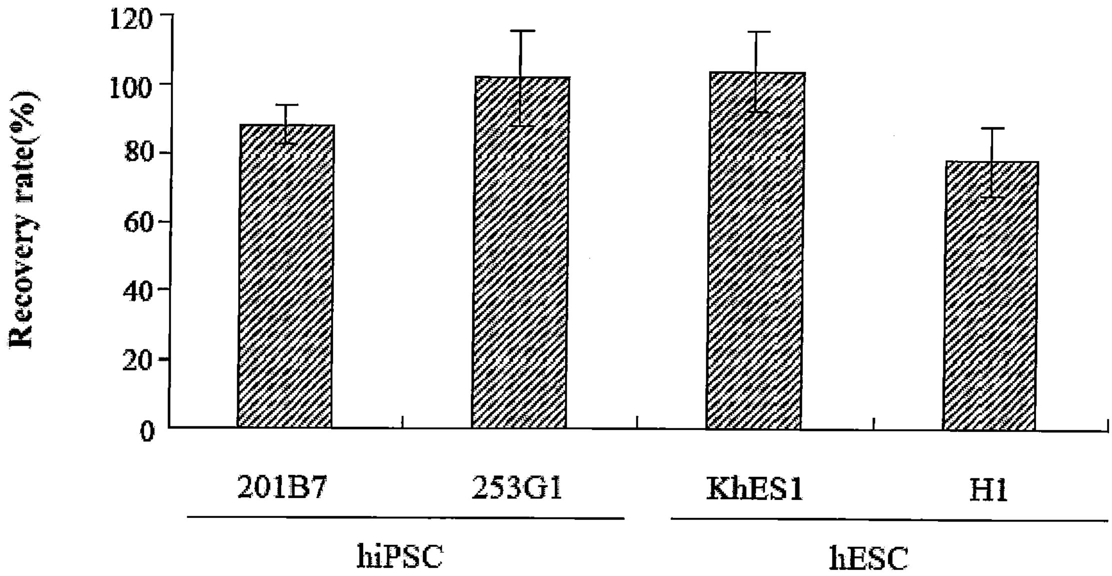
120
100
80
60
40
20
0
Recovery rate(%)
201B7
253G1
KhES1
H1
hiPSC
hESC

Fig.8

(1)

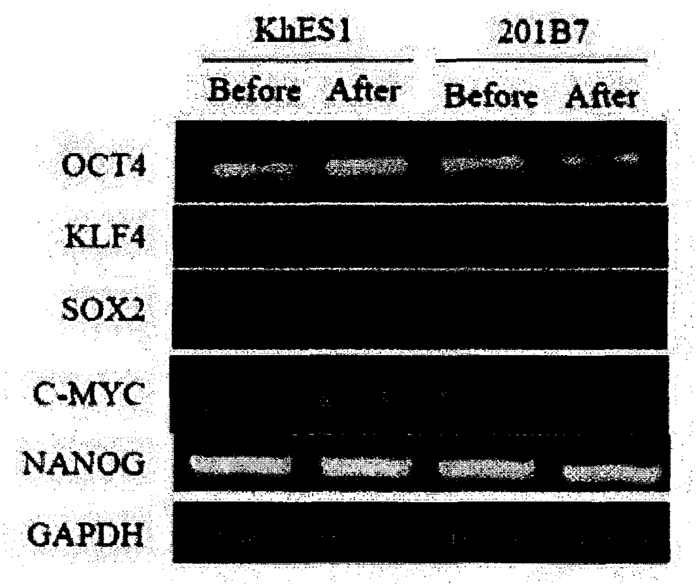
KhES1
201B7
Before
After
Before
After
OCT4
KLF4
SOX2
C-MYC
NANOG
GAPDH

(2)

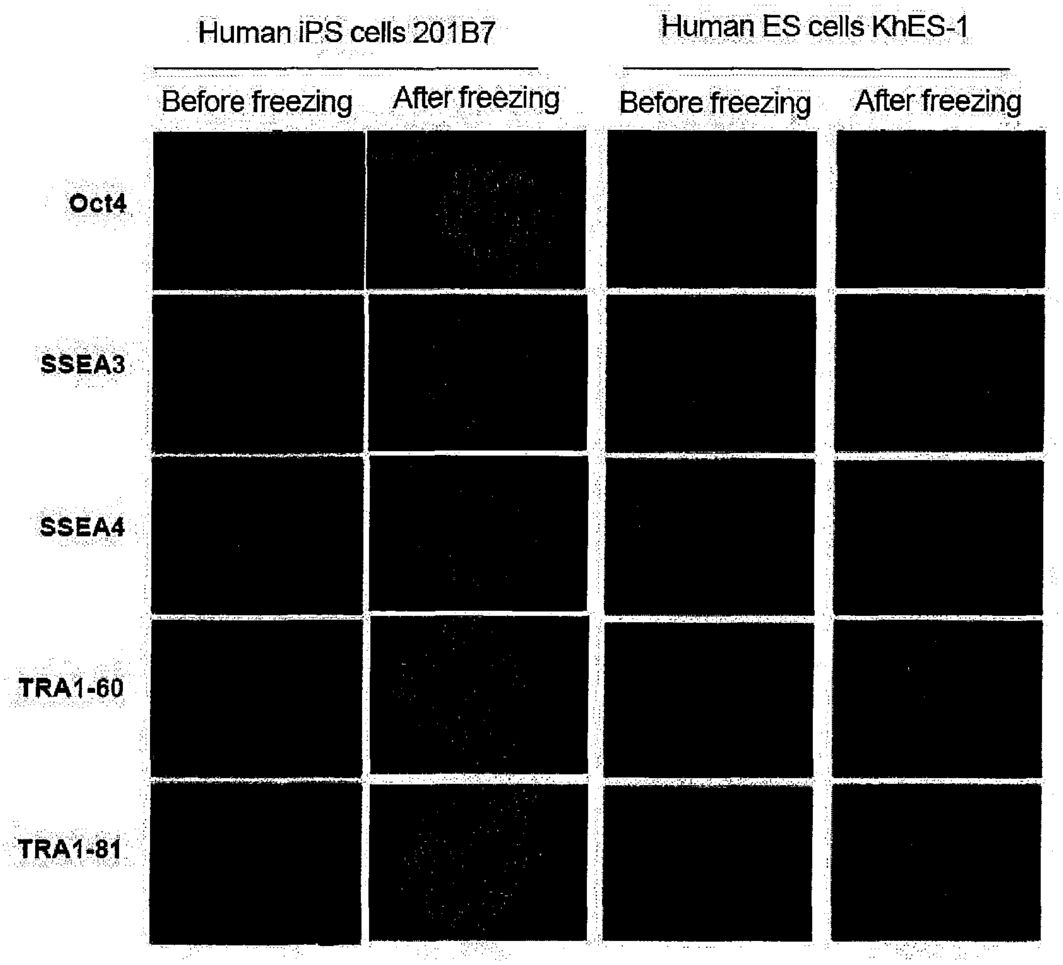
Human iPS cells 201B7
Human ES cells KhES-1
Before freezing
After freezing
Before freezing
After freezing
Oct4
SSEA3
SSEA4
TRA1-60
TRA1-81

Fig.9

(1)

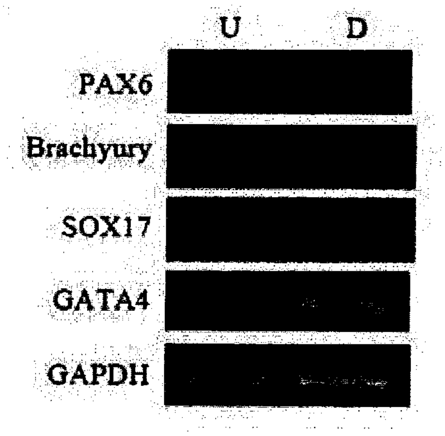
U
D
PAX6
Brachyury
SOX17
GATA4
GAPDH

(2)

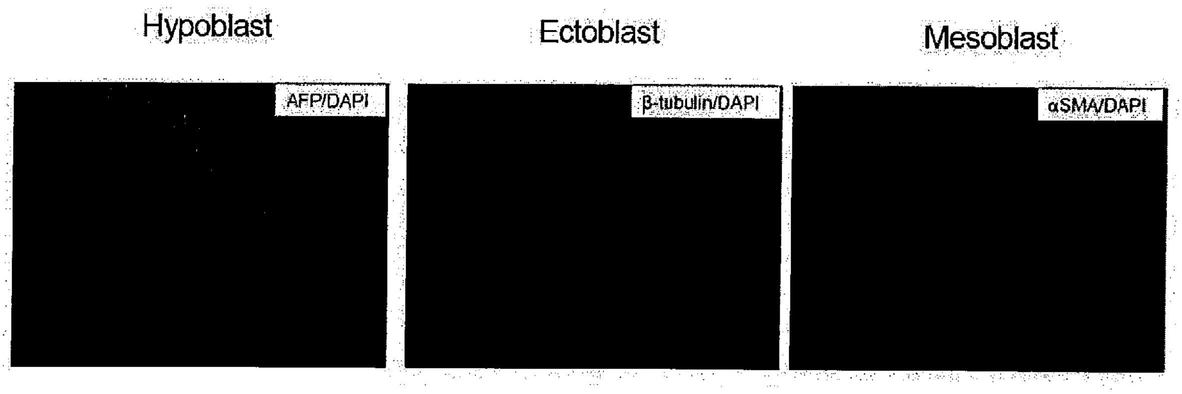
Hypoblast
Ectoblast
Mesoblast
AFP/DAPI
β-tubulin/DAPI
αSMA/DAPI

Fig.10

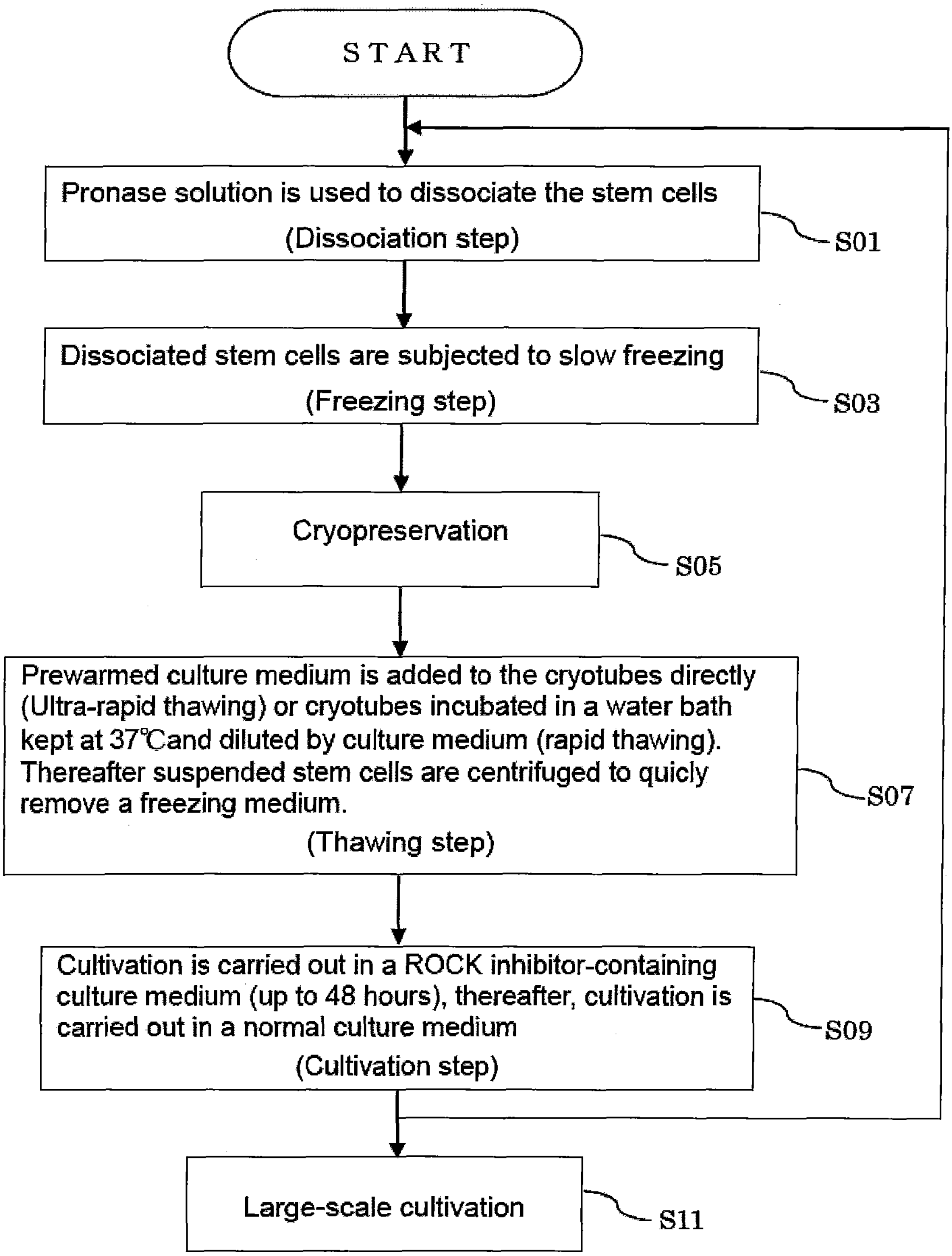
START
Pronase solution is used to dissociate the stem cells
(Dissociation step)
S01
Dissociated stem cells are subjected to slow freezing
(Freezing step)
S03
Cryopreservation
S05
Prewarmed culture medium is added to the cryotubes directly (Ultra-rapid thawing) or cryotubes incubated in a water bath kept at 37℃and diluted by culture medium (rapid thawing). Thereafter suspended stem cells are centrifuged to quicly remove a freezing medium.
(Thawing step)
S07
Cultivation is carried out in a ROCK inhibitor-containing culture medium (up to 48 hours), thereafter, cultivation is carried out in a normal culture medium
(Cultivation step)
S09
Large-scale cultivation
S11

Fig.11

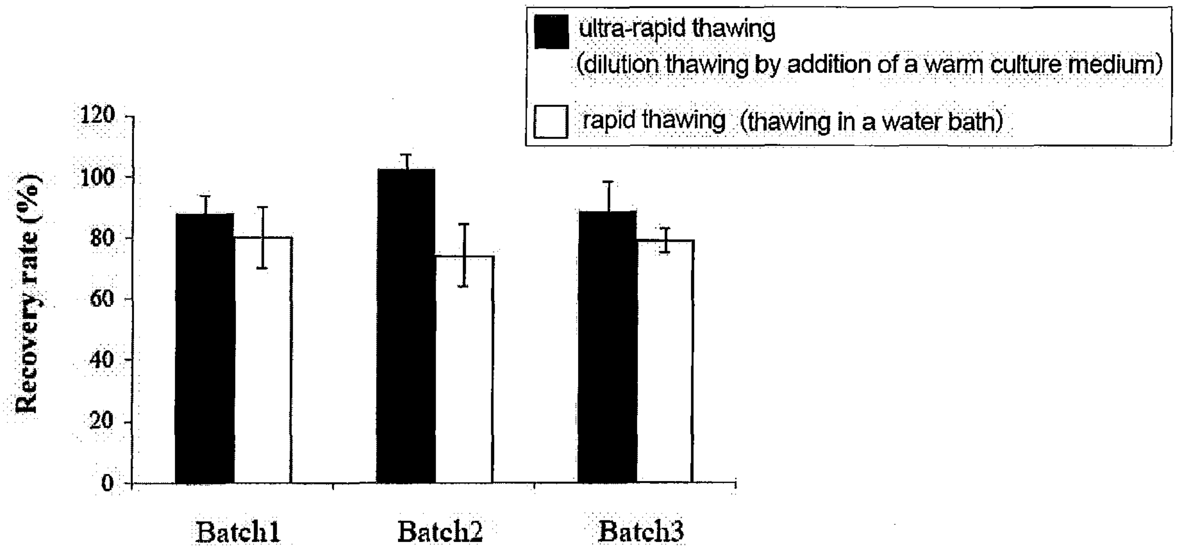
ultra-rapid thawing
(dilution thawing by addition of a warm culture medium)
rapid thawing (thawing in a water bath)
Recovery rate (%)
120
100
80
60
40
20
0
Batch1
Batch2
Batch3

Fig.12

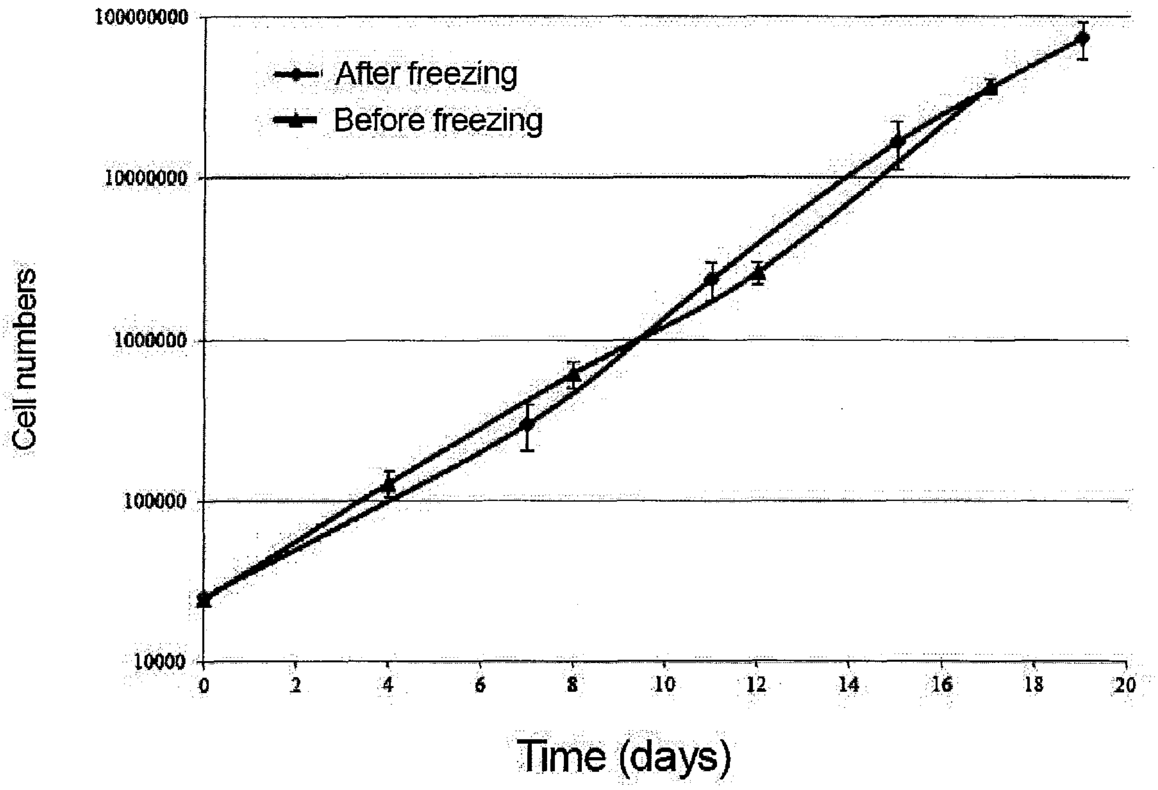
After freezing
Before freezing
Cell numbers
100000000
10000000
1000000
100000
10000
0
2
4
6
8
10
12
14
16
18
20
Time (days)

Fig.13

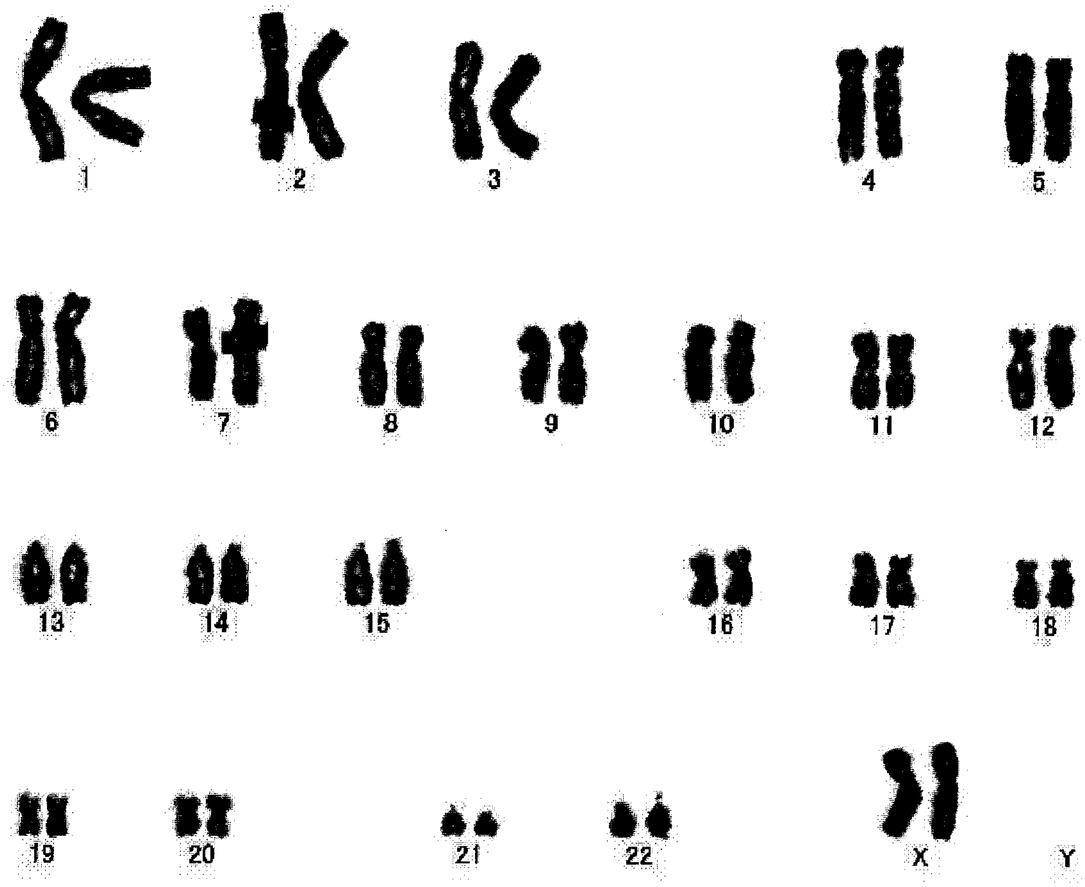

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012136422 A
- JP 2012136442 A

### Non-patent literature cited in the description


- *Human Reproduction,* 2005, vol. 20, 1779-1785
- *Reprod Biomed Online,* 2005, vol. 11, 733-739
- *Human Reproduction,* 2010, vol. 25, 1271-1279
- **T' JOEN et al.** *An efficient, economical slow-freezing method for large-scale human embryonic stem cell banking Stem Cells and Dev,* 2012, vol. 21, 721-8
- **WATANABE K et al.** *Nat Biotechnol,* 2007, vol. 25, 681-686
- **MARTIN-IBANEZ R et al.** *Human Reprod,* 2008, vol. 23, 2744-2754
- **MARTIN-IBANEZ R et al.** *Human Reprod,* 2009, vol. 24, 492-493
- **LI XY et al.** *Human Reprod,* 2009, vol. 24, 580-589
- **MOLLAMOHAMMADI S et al.** *Human Reprod,* vol. 24, 2468-2476
- **TAKENAKA et al.** *Experimental Hematology,* 2010, vol. 38 (2), 154-62

SEQUENCE LISTING

<110> Kyokuto Seiyaku Kogyo Kabushiki Kaisha; RIKEN
<120> A stem cell preservation medium, stem cell preservation methods, and stem cell preservation system
<140> JP2012-136442
<141> 2012-06-15
<160> 20
<210> 1
<211> 24
<212> RNA
<213> Artificial Sequence
<400> 1
gacaggggga ggggaggagc tagg 24

<210> 2
<211> 26
<212> RNA
<213> Artificial Sequence
<220>
<400> 2
cttccctcca accagttgcc ccaaac 26

<210> 3
<211> 30
<212> RNA
<213> Artificial Sequence
<220>
<400> 3
gattacgcgg gctgcggcaa aacctacaca 30

<210> 4
<211> 28
<212> RNA
<213> Artificial Sequence
<400> 4
tgattgtagt gctttctggc tgggctcc 28

<210> 5
<211> 26
<212> RNA
<213> Artificial Sequence
<400> 5
gggaaatggg aggggtgcaa aagagg 26

<210> 6
<211> 26
<212> RNA
<213> Artificial Sequence
<400> 6
ttgcgtgagt gtggatggga ttggtg 26

<210> 7
<211> 30

<212> RNA
<213> Artificial Sequence
<400> 7
tttctgaaga ggacttgttg cggaaacgac 30

<210> 8
<211> 30
<212> RNA
<213> Artificial Sequence
<400> 8
tcagccaagg ttgtgaggtt gcatttgatc 30

<210> 9
<211> 25
<212> RNA
<213> Artificial Sequence
<400> 9
cagccccgat tcttccacca gtccc 25

<210> 10
<211> 25
<212> RNA
<213> Artificial Sequence
<400> 10
cggaagattc ccagtcgggt tcacc 25

<210> 11
<211> 23
<212> RNA
<213> Artificial Sequence
<400> 11
cttcttttgc gtcgccagcc gag 23

<210> 12
<211> 22
<212> RNA
<213> Artificial Sequence
<400> 12
cagccttgac ggtgccatgg aa 22

<210> 13
<211> 28
<212> RNA
<213> Artificial Sequence
<400> 13
acccattatc cagatgtgtt tgcccgag 28

<210> 14
<211> 26
<212> RNA
<213> Artificial Sequence
<400> 14
atggtgaagc tgggcatagg cggcag 26

<210> 15
<211> 20
<212> RNA
<213> Artificial Sequence

<400> 15
gatgcagtga ctttttgtcg 20

<210> 16
<211> 20
<212> RNA
<213> Artificial Sequence
<400> 16
ctactgcagg tgtgagcaag 20

<210> 17
<211> 26
<212> RNA
<213> Artificial Sequence
<400> 17
cgctttcatg gtgtgggcta aggacg 26

<210> 18
<211> 26
<212> RNA
<213> Artificial Sequence
<400> 18
tagttggggt ggtcctgcat gtgctg 26

<210> 19
<211> 20
<212> RNA
<213> Artificial Sequence
<400> 19
ctagaccgtg ggttttgcat 20

<210> 20
<211> 20
<212> RNA
<213> Artificial Sequence
<400> 20
tgggttaagt gcccctgtag 20